(19) Europäisches Patentamt
European Patent Office
Office européen des brevets

(11) EP 4 527 395 A1

(12) EUROPEAN PATENT APPLICATION
published in accordance with Art. 153(4) EPC

(43) Date of publication:
26.03.2025 Bulletin 2025/13

(51) International Patent Classification (IPC):
A61K 35/30 (2015.01)          A61P 25/28 (2006.01)
A61P 19/10 (2006.01)          A61P 21/00 (2006.01)
A61P 43/00 (2006.01)          C12N 5/0797 (2010.01)

(21) Application number: 23807950.3

(22) Date of filing: 19.05.2023

(52) Cooperative Patent Classification (CPC):
A61K 35/30; A61P 19/10; A61P 21/00;
A61P 25/28; A61P 43/00; C12N 5/06

(86) International application number:
PCT/KR2023/006861

(87) International publication number:
WO 2023/224431 (23.11.2023 Gazette 2023/47)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 20.05.2022 KR 20220062408

(71) Applicant: CorestemChemon Inc.
Seongnam-si, Gyeonggi-do 13486 (KR)

(72) Inventors:
• KIM, Kyung Suk
Seoul 02838 (KR)
• LEE, Tae Yong
Yongin-si Gyeonggi-do 17128 (KR)
• KIM, Min Sung
Seoul 05771 (KR)
• LEE, Sang-Hun
Seoul 04717 (KR)
• SULISTIO, Yanuar Alan
Seoul 04792 (KR)
• KIM, Min Soo
Seongnam-si Gyeonggi-do 13508 (KR)

(74) Representative: HGF
HGF Limited
1 City Walk
Leeds LS11 9DX (GB)

Remarks:
The complete document including Reference
Table(s) and the Sequence Listing(s) can be
downloaded from the EPO website

(54) PHARMACEUTICAL COMPOSITION FOR PREVENTING AND TREATING GERIATRIC DISEASES COMPRISING NEURAL STEM CELLS DERIVED FROM THREE-DIMENSIONAL HYPOTHALAMUS ORGANOID AND USE THEREOF

(57) The present invention relates to a pharmaceutical composition for preventing and treating geriatric diseases, comprising hypothalamus-like organoid-derived neural stem cells as an active ingredient. It was found that decreased memory, cognitive ability, muscle strength, muscle tissue density, and bone mineral density in aging mice due to aging were restored when the hypothalamus-like organoid-derived neural stem cells according to the present invention were transplanted into the aging mice. In addition, the transplantation of the hypothalamus-like organoid-derived neural stem cells was found to improve obesity by increasing energy metabolism. It was found that decreased memory, cognitive ability, and muscle strength in aging mice were similarly restored when exosomes isolated from the hypothalamus-like organoid-derived neural stem cells were transplanted into the aging mice. Therefore, the hypothalamus-like organoid-derived neural stem cells according to the present invention are expected to be very useful as an agent for preventing and treating geriatric diseases.

EP 4 527 395 A1

[Figure 5]

**Description**

**Technical Field**

The present invention relates to a pharmaceutical composition for preventing and treating geriatric diseases, comprising neural stem cells obtained by isolating and dissociating hypothalamus-like organoids produced by 3D culture from human pluripotent stem cells as an active ingredient.

**Background Art**

The hypothalamus is a central organ for maintaining homeostasis in the human body, such as growth, puberty, metabolism, stress response, reproduction, lactation, childcare, and immunity. Neural stem/progenitor cells exist in the hypothalamus of adults and are known to regulate the function of the hypothalamus. Therefore, neural stem cells have recently been attracting attention as an alternative treatment for hypothalamic dysfunction caused by aging, etc.

However, since the hypothalamus is located deep in the brain and is a small area, it is impossible to extract actual tissue and use it for research. Therefore, research has been conducted steadily to produce hypothalamic neural cells from pluripotent stem cells such as embryonic stem cells. For example, Wataya's team produced neural cells that produce hypothalamus arginine vasopressin (AVP) from mouse embryonic stem cells through 3D suspension culture by aggregating cells using gfCDM medium (Wataya T et al., Proc Natl Acad Sci USA, 2008, 105(33):11796-11801). In addition, Merkle's team succeeded in differentiating from human embryonic stem cells and induced pluripotent stem cells into hypothalamic neural cells through 3D and 2D culture methods (Merkle FT et al., Development, 2015, 142(4):633-643). However, despite the above-mentioned diverse studies, there are still limitations in clinical application.

**Detailed Description of Invention**

**Technical Problem**

Accordingly, the present inventors found that neural stem cells obtained from hypothalamus-like organoids cultured in 3D from human embryonic stem cells are similar to neural stem cells existing in the hypothalamus of the actual brain, and that when transplanted into aging mice, symptoms related to aging were ameliorated. Based on the above, the present inventors completed the present invention.

**Solution to Problem**

In order to solve the above problem, in one aspect of the present invention, there is provided a pharmaceutical composition for preventing and treating geriatric diseases, comprising hypothalamus-like organoid-derived neural stem cells as an active ingredient.

In another aspect of the present invention, there is provided an exosome secreted from a hypothalamus-like organoid-derived neural stem cell; and a pharmaceutical composition for preventing and treating geriatric diseases, comprising the same as an active ingredient.

In another aspect of the present invention, there is provided a method for producing an exosome, comprising the steps of: i) culturing hypothalamus-like organoid-derived neural stem cells; and ii) collecting exosomes from the culture fluid.

In another aspect of the present invention, there is provided a kit for preventing and treating geriatric diseases, comprising a cell and/or an exosome selected from the group consisting of the hypothalamus-like organoid-derived neural stem cell; and an exosome secreted from the hypothalamus-like organoid-derived neural stem cell.

In another aspect of the present invention, there is provided a use of the hypothalamus-like organoid-derived neural stem cell; or an exosome secreted from the hypothalamus-like organoid-derived neural stem cell for the prevention or treatment of geriatric diseases.

In another aspect of the present invention, there is provided a method for preventing or treating geriatric diseases, comprising the step of administering to a subject the hypothalamus-like organoid-derived neural stem cell; or an exosome secreted from the hypothalamus-like organoid-derived neural stem cell.

**Effects of Invention**

It was found that decreased memory, cognitive ability, muscle strength, muscle tissue density, and bone mineral density due to aging were restored when neural stem cells derived from a hypothalamus-like organoid according to the present invention were transplanted into aging mice. In addition, the transplantation of the hypothalamus-like organoid-derived neural stem cells was found to ameliorate obesity by increasing energy metabolism. It was also found that decreased memory, cognitive ability, and muscle strength were similarly restored when exosomes isolated from a culture fluid of neural stem cells derived from a hypothalamus-like organoid were transplanted into aging mice. Therefore, the neural stem cells derived from a hypothalamus-like organoid and exosomes isolated from a culture fluid thereof are expected to be very useful as an agent for preventing and treating geriatric diseases.

**Brief Description of Drawings**

Figure 1 is a schematic diagram of a method for producing human hypothalamus organoids from human embryonic stem cells (hESCs) or human dedifferentiated stem cells (hiPSCs) and inducing differentiation from the produced hypothalamus organoids into hypothalamus neural stem cells (htNSCs).

Figure 2 is a graph showing the results of confirming the expression of hypothalamus (NKX2-1, RAX) or midbrain (EN1) marker genes by q-PCR after treating the culture fluid of organoids produced by culture of hESCs with various hypothalamus patterning-inducing substances.

Figure 3 is a diagram showing the results of confirming the expression of neural stem cell markers (SOX2, NESTIN, KI67) and hypothalamus region-specific marker genes (NKX2-1, RAX, ISL1) in organoid-derived human htNSCs by immunofluorescence staining. At this time, DAPI indicates cell nucleus.

Figure 4 is a graph showing the results of confirming the expression of hypothalamus region-specific marker genes (RAX, NKX2.1, ISL1, OTP, SF-1) in organoid-derived human htNSCs by q-PCR.

Figure 5 is a diagram showing the results of analyzing the transcript of organoid-derived human htNSCs and the transcript of each region of the human fetal brain by DGE (differential gene expression).

Figure 6 is a graph showing the population doubling level (PDL) and accumulated cell number by measuring the proliferation of cell numbers during five passage cultures of organoid-derived human htNSCs in the presence of bFGF (basic fibroblast growth factor).

Figure 7 is a diagram and graph showing the results of observing the expression of proteins related to cell death (ethidium homodimer I dye, EthD-1), cell survival (calcein), and neural stem cells (Rax, Nestin) by immunofluorescence staining after re-culturing cryopreserved organoid-derived human htNSCs.

Figure 8 is a diagram showing the results of confirming the expression of neural cell markers (synapsin1 (SYN1), TuJ1 (TUBB), MAP2, RBFOX3 (NeuN)), hypothalamus specific neuropeptides ($\alpha$-MSH, neuropeptide Y (NPY), secretagogin (SCGN), PMCH), and astrocyte markers (GFAP) in differentiated htNSCs by immunofluorescence staining when organoid-derived human htNSCs were induced to differentiate into neural cells.

Figure 9 is a diagram (top) showing the results of confirming STAT3 or FOXO1 phosphorylation by leptin or ghrelin treatment by Western blot after differentiation from organoid-derived human htNSCs into neural cells, and a graph (bottom) showing the results of quantification.

Figure 10 is a diagram showing the results of visualizing the results of clustering by performing single cell RNA sequencing by UMAP (Uniform Manifold Approximation and Projection) after inducing differentiation of organoid-derived human htNSCs for 70 days.

Figure 11 is a diagram showing the results of analyzing each cluster specific gene.

Figure 12 is a diagram showing the results of visualizing the results of analyzing each cluster specific gene by UMAP.

Figure 13 is a diagram showing the results of comparing and analyzing the clustering results with the mouse brain transcript (14 days old) of the Allen Developing Brain atlas using Voxhunt. As a result of the comparison, all cell clusters differentiated from htNSCs were precisely located in the hypothalamus of the mouse brain at 14 days old, confirming that the cells differentiated by this established protocol are hypothalamic-specific cells.

Figure 14 is a diagram visualizing the results of single cell RNA sequencing according to pseudotime using pseudotime analysis using Monocle3 after inducing differentiation of organoid-derived human htNSCs for 70 days.

Figure 15 shows that hypothalamus neural stem cells (htNSCs) differentiate from intermediate cells (Int1, Int2) into neural stem cells (NSCs) through pseudotime analysis, and that cell proliferation factor CDK1, pro-neural gene ASCL1, and neural cell factors TUBB3 and SNAP25 also change in expression according to each developmental stage.

Figure 16 is a graph showing the unique expression increase/decrease pattern results of ROBO1, SLIT1, and SLIT2, known as key factors in hypothalamus development, through pseudotime analysis when hypothalamus neural stem cells (htNSCs) differentiate from intermediate cells (Int1, Int2) into neural stem cells (NSCs).

Figure 17 is a graph showing the expression of the master regulator (regulon) of each cell cluster (Neu1, Ast1, Tan) by performing gene regulatory network (GRN) analysis using pySCENIC.

Figure 18 is a diagram showing the results of visualizing the results of clustering the neural cell cluster (Neu1 and Neu2) by reanalyzing it using t-SNE (t-distributed stochastic neighbor embedding).

Figure 19 is a diagram showing the expression distribution of hypothalamus-related genes for each reanalyzed neural cell cluster.

Figure 20 is a diagram showing the gene expression pattern of single cells for each cluster by reanalyzing the neural cell cluster (Neu1, Neu2).

Figure 21 is a diagram showing the results of visualizing the results of clustering the astrocyte progenitor cell cluster (APC) by UMAP.

Figure 22 is a diagram showing the expression distribution of GFAP, SPARCL1, and AQP4 genes for each cell cluster within the astrocyte progenitor cell cluster (APC).

Figure 23 is a diagram showing the expression pattern of single cell genes expressed for each cell cluster within the astrocyte cluster (Ast1, Ast2, Ast3), the astrocyte progenitor cell cluster (APC), and the tanycyte cluster (Tan).

Figure 24 is a diagram showing an experimental schedule for confirming the aging ameliorating effect in mice transplanted with organoid-derived human htNSCs.

Figure 25 is a diagram showing the results of observing the expression of human cell markers (hNCAM), hypothalamus neural stem cell markers (RAX), neural cell markers (RBFOX3 (NeuN)), and human astrocyte markers (hGFAP) in the transplanted cells in the hypothalamus of mice by immunofluorescence staining 1 month after transplanting organoid-derived human htNSCs into the hypothalamus of 12-month-old mice.

Figure 26 is a graph showing the results of measuring the treadmill performance of mice 1.5 months (13.5 months old) and 4 months (16 months old) after transplanting organoid-derived human htNSCs into the mouse hypothalamus.

Figure 27 is a graph showing the results of a grip strength test of mice 1.5 months (13.5 months old) and 4 months (16 months old) after transplanting organoid-derived human htNSCs into the mouse hypothalamus.

Figure 28 is a graph showing the results of a rotarod test of mice 1.5 months (13.5 months old) and 4 months (16 months old) after transplanting organoid-derived human htNSCs into the mouse hypothalamus.

Figure 29 is a graph showing the results of an open field test of mice 1.5 months (13.5 months old) and 4 months (16 months old) after transplanting organoid-derived human htNSCs into the mouse hypothalamus.

Figure 30 is a graph showing the time required for spatial perception learning during hidden platform training in the results of a Morris water maze test of mice 1.5 months (13.5 months old) and 4 months (16 months old) after transplanting organoid-derived human htNSCs into the mouse hypothalamus

Figure 31 is a graph showing the time spent in the target quadrant during the probe trial in the results of a Morris water maze test of mice 1.5 months (13.5 months old) and 4 months (16 months old) after transplanting organoid-derived human htNSCs into the mouse hypothalamus.

Figure 32 is a graph showing the results of a Y maze test of mice 1.5 months (13.5 months old) and 4 months (16 months old) after transplanting organoid-derived human htNSCs into the mouse hypothalamus.

Figure 33 is a graph showing the results of a novel object recognition test of mice 1.5 months (13.5 months old) and 4 months (16 months old) after transplanting organoid-derived human htNSCs into the mouse hypothalamus.

Figure 34 is a graph showing the results of measuring the body weight of mice and a diagram showing the results of observing the appearance of mice 1.5 months (13.5 months old) and 4 months (16 months old) after transplanting organoid-derived human htNSCs into the mouse hypothalamus.

Figure 35 is a graph showing the results of measuring the fat mass and lean mass of a mouse 4 months (16 months old) after transplanting organoid-derived human htNSCs into the mouse hypothalamus.

Figure 36 is a diagram showing the results of measuring the food intake of mice 1.5 months (13.5 months old) and 4 months (16 months old) after transplanting organoid-derived human htNSCs into the mouse hypothalamus.

Figure 37 is a graph showing the results of measuring the locomotor activity of mice using a metabolic cage 1.5 months (13.5 months old) and 4 months (16 months old) after transplanting organoid-derived human htNSCs into the mouse hypothalamus.

Figure 38 is a graph showing the results of measuring the energy expenditure of mice using a metabolic cage 1.5 months (13.5 months old) and 4 months (16 months old) after transplanting organoid-derived human htNSCs into the mouse hypothalamus.

Figure 39 is a diagram showing the results of a glucose tolerance test of a mouse 4 months (16 months old) after transplanting organoid-derived human htNSCs into the mouse hypothalamus.

Figure 40 is a diagram (left) showing the results of H&E staining of the inguinal white adipose tissue (iWAT) of a mouse and a graph (right) showing the results of measuring the size of adipocytes of a mouse 4 months (16 months old) after transplanting organoid-derived human htNSCs into the mouse hypothalamus.

Figure 41 is a diagram (left) showing the results of H&E staining of the epididymal white adipose tissue (eWAT) of a mouse and a graph (right) showing the results of measuring the size of adipocytes of a mouse 4 months (16 months old) after transplanting organoid-derived human htNSCs into the mouse hypothalamus.

Figure 42 is a diagram (left) showing the results of H&E staining of the interscapular brown adipose tissue (iBAT) of a mouse and a graph (right) showing the results of measuring the size of adipocytes of a mouse 4 months (16 months old) after transplanting organoid-derived human htNSCs into the mouse hypothalamus.

Figure 43 is a diagram showing the results of H&E staining of the liver tissue of a mouse 4 months (16 months old) after transplanting organoid-derived human htNSCs into the mouse hypothalamus. It was confirmed that fat accumulation in the liver tissue was reduced in the htNSC-transplanted mouse.

Figure 44 is a diagram (top) showing the results of confirming the expression of β-actin and proteins (MTCO1, SDHB, FGF21) related to lipolysis, thermogenesis, and browning in the inguinal white adipose tissue (iWAT) of a mouse by Western blot, and a graph (bottom) showing the results of quantifying the expression with β-actin, 4 months (16 months old) after transplanting organoid-derived human htNSCs into the mouse hypothalamus.

Figure 45 is a diagram (top) showing the results of confirming the expression of β-actin and proteins (MTCO1, SDHB, FGF21) related to lipolysis, thermogenesis, and browning in the epididymal white adipose tissue (eWAT) of a mouse by Western blot, and a graph (bottom) showing the results of quantifying the expression with β-actin, 4 months (16 months old) after transplanting organoid-derived human htNSCs into the mouse hypothalamus.

Figure 46 is a diagram (top) showing the results of confirming the expression of β-actin and proteins (p-eIF2A, eIF2A, ATF4, FGF21, GDF15) related to lipolysis and stress in the liver tissue of a mouse by Western blot, and a graph (bottom) showing the results of quantifying the expression with β-actin, 4 months (16 months old) after transplanting organoid-derived human htNSCs into the mouse hypothalamus.

Figure 47 is a diagram (top) showing the results of confirming the expression of p16 and p21 in the kidney tissue of a mouse by Western blot, and a graph (bottom) showing the results of quantifying the expression, 4 months (16 months old) after transplanting organoid-derived human htNSCs into the mouse hypothalamus.

Figure 48 is a diagram (top) showing the results of X-ray imaging of the femur of a mouse and a graph (bottom) showing the results of bone index analysis of a mouse 4 months (16 months old) after transplanting organoid-derived human htNSCs into the mouse hypothalamus. BMD: bone mineral density, BV: bone volume, TV: tissue volume, Tb.Th: trabecular thickness, Tb.N: trabecular number

Figure 49 is a schematic diagram of an experimental schedule to confirm the anti-aging effect of organoid-derived human htNSC-derived exosomes.

Figure 50 is a graph showing the results of a rotarod test of mice 1.5 months (13.5 months old) and 4 months (16 months old) after the injection of organoid-derived human htNSC-derived exosomes.

Figure 51 is a graph showing the results of a grip strength test of mice 1.5 months (13.5 months old) and 4 months (16 months old) after the injection of organoid-derived human htNSC-derived exosomes.

Figure 52 is a graph showing the results of a passive avoidance test 1.5 months (13.5 months old) and 4 months (16 months old) after the injection of organoid-derived human htNSC-derived exosomes.

Figure 53 is a graph showing the time required for spatial perception learning during hidden platform training and the time spent in the target quadrant during the probe trial in the results of a Morris water maze test of mice 1.5 months (13.5 months old) and 4 months (16 months old) after the injection of organoid-derived human htNSC-derived exosomes.

## Best Mode for Carrying out the Invention

## Pharmaceutical composition comprising neural stem cell

In one aspect of the present invention, there is provided a pharmaceutical composition comprising hypothalamus-like organoid-derived neural stem cells as an active ingredient. In addition, the pharmaceutical composition may be used for preventing and treating geriatric diseases.

At this time, the pharmaceutical composition may comprise hypothalamus-like organoid-derived neural cells; or a cell population comprising hypothalamus-like organoid-derived neural stem cells and hypothalamus-like organoid-derived neural cells as an active ingredient.

As used herein, the term "hypothalamus" is located below the two thalamuses and functions to connect the nervous system and the endocrine system via the pituitary gland. Specifically, the hypothalamus controls metabolic processes and the activities of the autonomic nervous system, and synthesizes and secretes neurohormones to regulate the pituitary gland, thereby regulating body temperature, hunger, thirst, fatigue, sleep, and circadian rhythms.

As used herein, the term "organoid" refers to a 'miniaturized organ-like structure' produced using stem cells to have a minimum function, and is characterized in that it is made in a three-dimensional (3D) structure and can create an environment similar to an actual body organ even in a laboratory. In other words, the organoid refers to a cell having a 3D stereostructure, and refers to a model similar to organs such as nerves and intestines produced through an artificial culture process that has not been collected or acquired from animals and the like. At this time, the origin of the cells constituting it is not limited.

In addition, the organoid may have an environment that is allowed to interact with the surrounding environment in the process of cell growth. Unlike 2D culture, 3D cell culture allows cells to grow in all directions *ex vivo*. Accordingly, in the present invention, the 3D organoid can be an excellent model for observing the development of therapeutic agents for diseases and the like by almost completely mimicking the organs that actually interact *in vivo.*

In general, the organoid may be produced by culturing pluripotent stem cells. At this time, pluripotent stem cells (PSCs) refer to stem cells that can be induced to differentiate into any type of cell that constitutes the body, and may include embryonic stem cells and induced pluripotent stem cells (iPSCs, dedifferentiated stem cells), but are not limited thereto. The pluripotent stem cells may be isolated from mammals, but may be preferably isolated from human.

In the present invention, the organoid may be a hypothalamus-like organoid that mimics the hypothalamus.

Specifically, in the present invention, the hypothalamus-like organoid may be produced by proliferating and culturing human embryonic stem cells (hESCs) to produce a 3D nervous system organoid and treating it with a substance that induces patterning toward the hypothalamus region. The patterned hypothalamus-like organoid may be dissociated to ultimately yield hypothalamus neural stem cells.

As used herein, the term "patterning" means producing an organoid such that a cell cluster of the fate having the characteristics of the origin tissue of the cell to be ultimately extracted among the specific tissues in the brain is contained as the majority of all clusters (target cell enriched) in producing an organoid.

In the present invention, the patterning may be patterning toward the hypothalamus region.

In the present invention, the production and patterning of nervous system organoids from human pluripotent stem cells can be achieved by culturing in a medium containing ascorbic acid, an ALK inhibitor, and a BMP inhibitor. Preferably, the ALK inhibitor may be SB431542, and the BMP inhibitor may be noggin.

In addition, the material that induces patterning may additionally include a material that activates the hedgehog signal pathway. The material that activates the hedgehog signal pathway may be a sonic hedgehog (SHH) and an SMO (smoothened) agonist.

As used herein, the term "sonic hedgehog (SHH)" plays a very important role in cell proliferation and morphogenesis in the development of various body tissues such as the skeletal system, the central nervous system, and the face. In particular, it is a protein that plays an important role in the development of the central nervous system such as the spinal cord, cerebrum, and cerebellum, and in the proliferation and differentiation of neural stem cells. When the SHH binds to the cell membrane receptor PTCH1 (Patched1), the SMO receptor is activated, which inhibits SUFU (suppressor of fused), a negative regulator of hedgehog, thereby activating the transcription factor GLI1 and promoting the proliferation of neural cells. In the present invention, the SMO agonist may be purmorphamine.

The hypothalamus patterned organoids may be further cultured in a medium containing insulin, ascorbic acid, and bFGF.

As used herein, the term "medium" means a medium that allows support of the proliferation, survival, and differentiation of organoids *in vitro,* and includes all conventional media suitable for the culture and differentiation of organoids used in the pertinent field. The type of medium and culture conditions may be appropriately selected depending on the type of cells. In the present invention, the medium may be a medium suitable for inducing differentiation from human stem cells into neural progenitor cells.

As used herein, the term "neural stem cell (NSC)" means an immature cell that is capable of self-renewal and continues to proliferate in an undifferentiated state, and has the potential to differentiate into nervous system cells. The neural stem cell exists in various anatomical parts throughout the fetal nervous system of mammals including humans, and recently, the neural stem cells exist not only in the fetal but also in specific parts of the adult nervous system, and throughout life, the neural stem cells can continue to proliferate in specific parts of the brain and generate new neural cells. The neural stem cell can differentiate into neurons, astrocytes, and oligodendrocytes. The neural stem cells can be proliferated *in vitro* and transplanted *in vivo,* and can migrate, engraft, and integrate into the host nervous system to secrete therapeutically useful substances and differentiate into neural cells that are appropriate in terms of cell structure and function.

In the present invention, the hypothalamus-like organoid-derived neural stem cell (hypothalamic NSC, htNSC) may be characterized by expressing any one neural stem cell marker selected from the group consisting of SOX2, NESTIN, and KI67, and expressing any one hypothalamus marker selected from the group consisting of RAX, NKX2-1, ISL1, OTP, and SF-1. At this time, the hypothalamus-like organoid-derived neural stem cell may be characterized by expressing the neural stem cell marker and the hypothalamus marker at about 84% or more in the entire DAPI-positive cell population. At this time, the neural stem cell marker and the hypothalamus marker may be expressed at about 85%, about 86%, about 87%, about 88%, about 89%, about 90%, about 91%, about 92%, about 93%, about 94%, about 95%, about 96%, about 97%, about 98%, about 99% in the entire DAPI-positive cell population.

SOX2 (SRY-box transcription factor 2) is a transcription factor that regulates the pluripotency of embryonic stem cells, and plays a role in maintaining the self-renewal or pluripotency of embryonic stem cells and regulating the differentiation process into specific cells.

Nestin is a type VI intermediate filament (IF) protein that is mainly expressed in neural cells associated with the radial growth of axons. It is expressed in the early stages of development of the central nervous system, peripheral nervous system, muscle, and other tissues, and in adults, its expression is induced in pathological situations such as the formation of a glial scar after central nervous system injury and the regeneration of damaged muscle tissue. Nestin is known to play a role in regulating the assembly and disassembly of intermediate filaments involved in cell remodeling in mitotic cells.

KI67 is a nuclear protein associated with cell proliferation, and its expression is significantly increased in the S phase of the cell cycle, so it is used as a cell proliferation marker.

In addition, RAX (retina and anterior neural fold homeobox) is a transcription factor containing a paired-homeodomain, and is expressed in the anterior neural plate during early embryogenesis, and in the retina, hypothalamus, and pineal gland during late embryogenesis.

NKX2-1 is a transcription factor also known as thyroid transcription factor 1 (TTF-1) that regulates the expression of genes specific to the thyroid, lung, and diencephalon.

ISL1 (ISL LIM homeobox 1) is a transcription factor containing two LIM domains (N-terminal) and one homeodomain (C-terminal). ISL1 is known to regulate the development of the islets of Langerhans during embryogenesis and play an important role in the differentiation of motor neurons in the neural tube in mouse embryos.

OTP (orthopedia homeobox) is a transcription factor containing a homeodomain and is known to be involved in the differentiation and development of hypothalamic neuroendocrine cells.

SF-1 (steroidogenic factor 1) is known to be an essential factor for the development and function of the adrenal gland and reproductive organs. In the brain, it is known to be expressed only in the ventromedial nucleus of the hypothalamus (VMH), regulating VMH development and function and participating in energy metabolism homeostasis regulation.

In addition, the hypothalamus-like organoid-derived neural stem cells can maintain their morphology even after freezing and thawing, and can exhibit a high survival rate.

In addition, the hypothalamus-like organoid-derived neural stem cells can be passaged. Specifically, the hypothalamus-like organoid-derived neural stem cells can be passaged 1 to 10 times, 2 to 8 times, 3 to 7 times, or 4 to 6 times.

The hypothalamus-like organoid-derived neural stem cells according to the present invention can be differentiated into hypothalamus-specific neural cells.

Specifically, the hypothalamus-like organoid-derived neural stem cells according to the present invention can be differentiated into neural cells that express at least one neural cell marker selected from the group consisting of synapsin1 (SYN1), TuJ1 (TUBB), MAP2, and RBFOX3 (NeuN), and express at least one hypothalamus neuropeptide selected from the group consisting of $\alpha$-MSH, neuropeptide Y (NPY), secretagogin (SCGN), and PMCH.

Tuj1 is known as neuron-specific class III $\beta$-tubulin (TUBB3) and is a major component of microtubules. It is mainly expressed in neurons and is involved in neurogenesis and axon guidance and maintenance.

MAP2 (microtubule associated protein 2) is a cytoskeletal protein known to be involved in microtubule assembly, an essential step in neurogenesis. During the developmental stages of mouse neurons, MAP2 is mainly located in dendrites and is known to be involved in stabilizing the shape of dendrites.

Synapsin is a member of the synapsin family and functions in synapse formation and neurotransmitter release regulation.

NeuN is known as hexaribonucleotide binding protein-3 or Fox-3, and is a protein present in the nucleus of neurons that is generally used as a biomarker of neurons.

$\alpha$-MSH ($\alpha$-melanocyte-stimulating hormone) is an endogenous peptide hormone and a neuropeptide of the melanocortin family, and is one of the melanocyte-stimulating hormones that promotes melanin production. It is also known to be involved in feeding behavior, energy homeostasis, sexual behavior, and protection against ischemia and reperfusion injury.

NPY (neuropeptide Y) is a neuropeptide consisting of 36 amino acids that is involved in various physiological and homeostatic regulation in the central and peripheral nervous systems. NPY is the most abundant peptide in the central nervous system of mammals, which consists of the brain and spinal cord. In the autonomic nervous system, it is mainly produced by neurons of the sympathetic nervous system, plays a role in vasoconstriction, and is also involved in the growth of adipose tissue. In addition, it is known to be produced in various locations in the brain, including the hypothalamus, to increase food intake and energy storage, reduce anxiety, stress and pain sensitivity, and participate in regulating circadian rhythms.

Secretagogin (SCGN) is a calcium-binding protein expressed in the cytoplasm, and is known to be involved in calcium influx induced by potassium chloride and cell proliferation. In addition, it is known to be involved in the release of corticotropin releasing hormone (CRH), a stress hormone, and stress activation of the brain.

PMCH (Pro-Melanin Concentrating Hormone) is a precursor protein of MCH (Melanin Concentrating Hormone), and the MHC is a neuropeptide that regulates hunger, energy homeostasis, reproductive function, and sleep. It is known that dysfunction of PMCH is related to frontometaphyseal dysplasia 2 and Huntington's disease.

As used herein, the term "differentiation" refers to a phenomenon in which the structure or function of a cell becomes specialized while the cell divides and proliferates and the entire organism grows. In other words, it refers to a process in which cells, tissues, and the like of a living organism change to have a form and function suitable for performing their respective roles. In the present invention, the above term may include the process in which pluripotent stem cells transform into cells constituting the hypothalamus, and may also include the process in which progenitor cells express specific differentiation traits. In the present invention, when the hypothalamus-like organoid-derived neural stem cells (htNSCs) are differentiated into neural cells, astrocytes (GFAP⁺) and glial cells can also be differentiated together. At this time, the differentiated neural cells and astrocytes can sense the nutritional status of the body by responding to leptin and ghrelin, which are peptides produced in peripheral organs.

Specifically, the hypothalamus-like organoid-derived neural stem cell (htNSC) according to the present invention may be differentiated and clustered into a neural stem/progenitor cell cluster (NSC), an intermediate progenitor cell cluster (IPC), a neural cell cluster (Neu1 and Neu2), an astrocyte progenitor cell cluster (APC), an astrocyte cluster (Ast1, Ast2 and Ast3), a tanycyte cluster (Tan), an interneuron progenitor cell cluster (Int1 and Int2), a radial glia cluster (RG), and a proteoglycan-secreting glia cluster.

A progenitor cell is a cell that differentiates into a specific type of cell, and refers to a cell that differentiates into a "target cell" more specifically than a stem cell. While stem cells can infinitely self-divide, progenitor cells can divide a limited number of times. In the present invention, the progenitor cell may be a neural progenitor cell, an astrocyte progenitor cell, or an interneuron progenitor cell.

A neural cell is a cell that constitutes the nervous system and can transmit signals in an electrical manner using ion channels such as sodium channels and potassium channels. In addition, it receives and stores various information by exchanging chemical signals with other adjacent neural cells through structures called synapses. In the present specification, a neural cell may be used interchangeably with "neuron" or "neuron cell."

A neural progenitor cell refers to a cell that can differentiate into a neural cell.

In addition, an astrocyte is also referred to as astroglia and exists in the brain and spinal cord. It biochemically helps the cells inside the blood-brain barrier (BBB) and has projections attached to the blood vessel walls to supply nutrients to neural cells. It also performs various roles such as regulating ion concentrations in neural cells, supporting neural cells, removing waste products, and phagocytosis. In addition, when neural tissue is damaged, it proliferates into a projection referred to as glioma to fill the area and plays a role in repairing or destroying damaged tissue. It has been revealed that hydrogen peroxide generated during the process of astrocytes decomposing toxins is the cause of neural cell destruction, suggesting the possibility of dealing with brain diseases such as dementia.

An astrocyte progenitor cell refers to a cell that can differentiate into an astrocyte.

A tanycyte is an ependymal cell that exists in the third and fourth ventricles of the hypothalamus, and it senses nutrient status to regulate appetite.

An interneuron is a neural cell that connects two different areas of the brain, and is a type of nerve cell that is neither a sensory neuron nor a motor neuron. Sensory neurons transmit stimuli received by sensory organs to interneurons, which integrate and determine these stimuli and transmit them to motor neurons. Interneurons are included in the central nervous system and play a key role in neurogenesis in the mammalian adult brain, including reflexes and nerve vibrations.

An interneuron cluster (interneuron progenitor cell) refers to a cell that differentiates into an interneuron.

A radial glia is a type of glial cells and refers to a progenitor cell of neural cells and astrocytes. The radial glia also serves as a support to guide neural cells to the necessary location.

A proteoglycan-secreting glia is a glial cell that secretes proteoglycans. When the nervous system is damaged, astrocytes and mesenchymal cells secrete proteoglycans and extracellular matrix proteins to restore the damaged nervous system. This has a positive function of isolating the damaged area and preventing the spread of damage, but the substances secreted by astrocytes during the process of forming this glial scar also inhibit the growth of neurites.

Among the above clustered cell cluster, the neural cell cluster (Neu1 and Neu2) can express genes related to hypothalamus development. The astrocyte progenitor cell cluster (APC) may include cells that are differentiated into a cell cluster (Ast2) expressing at least one gene selected from the group consisting of SLC1A3, SLCO1C1, CRYM, and SCG2; a cell cluster (Ast1) expressing at least one gene selected from the group consisting of RSAD2, TRAIL, ISG5, IFITs, and STAT1; and a cell cluster (Ast3) expressing at least one gene selected from the group consisting of SOX9, ID3, Meis2, CLDN5, ALCAM, and COL1A2. At this time, the Ast2 cluster may be responsible for the nutritional supply and neuroendocrine function of neural cells. The Ast1 cluster may be responsible for an immune function. The Ast3 cluster may regulate the activity of neural cells. The tanycyte cluster (Tan) may be a cell cluster expressing at least one gene selected from the group consisting of RAX, FGF10, COL25A1, CRYM, and SCN7A.

SLC1A3 (solute carrier family 1 member 3) is a gene encoding excitatory amino acid transporter 1.

SLCO1C1 (solute carrier organic anion transporter family member 1C1) is a gene encoding a membrane transport protein (organic-anion-transporting polypeptides) that mediates the transport of organic anions, expressed in the brain and testis.

CRYM is a gene encoding Crystallin Mu homolog, also known as NADP-regulated thyroid-hormone-binding protein (THBP).

SCG2 is a gene encoding secretogranin II, a component protein of the chromogranin/secretogranin family of neuroendocrine proteins. Secretogranin II, when in its full-length form before cleavage, functions to sort and pack neuropeptides and peptide hormones into secretory bodies, and after cleavage, it functions as an activated secretoneurin.

RSAD2 is a gene encoding radical s-adenosyl methionine domain containing 2, also known as Viperin (virus inhibitory protein, endoplasmic reticulum-associated, interferon-inducible). Viperin is activated by interferon (IFN) to inhibit infection by DNA or RNA viruses.

TRAIL is a gene encoding TNF-related apoptosis-inducing ligand, a ligand that induces apoptosis.

IFIT is a gene encoding an interferon-induced antiviral protein (interferon induced protein with tetratricopeptide repeats).

STAT1 is a gene encoding a protein that mediates cell signaling for interferons, cytokines, and growth factors (signal transducer and activator of transcription 1).

SOX9 is a gene encoding a transcription factor that regulates chondrocyte differentiation and skeletal development.

ID3 is a gene encoding a transcription regulator that lacks a DNA binding site and forms a heterodimer to inhibit DNA binding and transcriptional activity. The transcription factor ID3 regulates various cellular processes including cell growth, senescence, differentiation, apoptosis, angiogenesis, and neoplastic transformation.

Meis2 is a gene encoding the homeobox protein Meis2, which binds to HOX or PBX proteins to form dimers or binds to DNA-binding dimers of PBX and HOX proteins to stabilize homoprotein-DNA complexes, thereby regulating transcription.

CLDN5 is a gene encoding Claudin-5, which specifically removes tight junctions within cells.

ALCAM is a gene encoding CD166, which mediates adhesion of thymic epithelial cells and CD6+ cells during T cell development in the thymus.

COL1A2 is a gene encoding collagen alpha-2(I) chain, a type of type 1 collagen.

FGF10 is a gene encoding fibroblast growth factor 10, which is involved in embryonic development, cell proliferation, cell differentiation regulation, and wound healing.

COL25A1 is a gene encoding collagen alpha-1(XXV) chain, which is a membrane-bound collagen protein that is expressed specifically in the brain and plays a role in inhibiting the fibrilization of amyloid-$\beta$ peptide (A$\beta$).

SCN7A is a gene encoding sodium channel protein type 7 subunit alpha. SCN7A plays a role in mediating voltage-dependent sodium ion permeability of excitable membranes.

**Use of pharmaceutical composition comprising neural stem cells**

A pharmaceutical composition comprising the hypothalamus-like organoid-derived neural stem cells according to the present invention as an active ingredient may be used for the purpose of preventing or treating geriatric diseases. In addition, the pharmaceutical composition may further comprise hypothalamus-like organoid-derived neural cells, or a cell population comprising neural stem cells and neural cells. At this time, the hypothalamus, the organoid, the neural stem cell, and the neural cell are the same as described above.

In the present invention, the geriatric disease may be selected from the group consisting of a neurodegenerative disease, osteoporosis, and a muscle aging-related disease.

At this time, the neurodegenerative disease may be selected from the group consisting of Parkinson's disease, dementia, Alzheimer's disease, Huntington's disease, amyotrophic lateral sclerosis, memory loss, myasthenia gravis, progressive supranuclear palsy, multiple system atrophy, essential tremor, corticobasal degeneration, diffuse Lewy body disease, and Pick disease.

At this time, the muscle aging-related disease may be selected from the group consisting of muscular atrophy, muscle disuse atrophy, and senile sarcopenia.

As used herein, the term "prevention" refers to any action of inhibiting or delaying the onset of a geriatric disease by administration of the pharmaceutical composition. The term "treatment" refers to any action in which symptoms of a geriatric disease are improved or beneficially changed by administration of the pharmaceutical composition.

In the pharmaceutical composition for preventing and treating geriatric diseases of the present invention, the neural stem cells, the neural cells, or a cell population comprising them may be included in any amount (effective amount) depending on the purpose, formulation, mixing purpose, and the like, as long as they can exhibit anti-aging activity. The pharmaceutical composition of the present invention may comprise about $2\times10^5$ to about $2\times10^{10}$ cells, preferably about $2\times10^6$ to about $2\times10^9$ cells, more preferably about $1\times10^7$ to about $2\times10^8$ cells, per 1 mL. Preferably, the pharmaceutical composition may comprise $2\times10^8$ cells per 1 mL.

Here, the "effective amount" refers to an amount of an active ingredient capable of inducing an anti-aging effect. Such an effective amount can be experimentally determined within the normal capabilities of those of ordinary skill in the art.

The composition of the present invention may be used unfrozen or may be frozen for later use. If frozen, standard cryopreservatives (e.g., DMSO, glycerol, polyvinylpyrrolidone, polyethylene glycol, albumin, dextran, sucrose, ethylene glycol, i-d erythritol, D-ribitol, D-mannitol, D-sorbitol, i-inositol, D-lactose, choline chloride, Epilife® cell freezing medium (Cascade Biologics)) may be added to the cell population prior to freezing.

The pharmaceutical composition of the present invention may be applied in the form of a cell therapeutic agent and may be formulated by further comprising a pharmaceutically acceptable carrier.

At this time, the "cell therapeutic agent" is a drug used for the purpose of treatment, diagnosis, and prevention with cells and tissues prepared through isolation from a subject, culture, and special fabrication (U.S. FDA regulations). It refers to a drug used for the purpose of treatment, diagnosis, and prevention through a series of actions such as proliferating or sorting living autologous, allogeneic, or xenogeneic cells *ex vivo*, or otherwise changing the biological properties of cells to restore the function of cells or tissues. The cell therapeutic agents are largely classified into a somatic cell therapeutic agent and a stem cell therapeutic agent depending on the degree of cell differentiation. In the present invention, the cell therapeutic agent may be a stem cell therapeutic agent.

As used herein, the term "pharmaceutically acceptable carrier" refers to a carrier or diluent that does not significantly stimulate an organism and does not inhibit the biological activity and properties of the administered ingredient. In the present invention, the pharmaceutically acceptable carrier that can be included in a cell therapeutic agent may be used without limitation as long as it is known in the art, such as a buffering agent, a preservative, an analgesic agent, a solubilizer, an isotonic agent, a stabilizer, a base, an excipient, a lubricant, and the like. The pharmaceutical composition of the present invention may be prepared in the form of various formulations according to commonly used techniques.

The pharmaceutical composition of the present invention can be administered in a pharmaceutically effective amount, and can be administered through any route as long as it can induce migration to the diseased site. Here, "administration" means introducing a predetermined substance to a subject by an appropriate method. In some cases, it may be considered that the pharmaceutical composition of the present invention is loaded into a vehicle comprising a means for directing neural stem cells to a lesion.

Therefore, the pharmaceutical composition of the present invention may be administered through several routes including topical (including buccal, sublingual, dermal and intraocular administration), parenteral (including subcutaneous, intradermal, intramuscular, instillation, intravenous, intraarterial, intraarticular and intracerebrospinal fluid) or transdermal administration, and preferably, may be administered directly to the site of disease. In one embodiment, the neural stem cells may be suspended in a suitable diluent and administered to a subject, and the diluent is used to protect and maintain the cells and to facilitate their use when injected into the target tissue. The diluent may include a buffer solution such as physiological saline, a phosphate buffer solution, and HBSS, cerebrospinal fluid, and the like. In addition, the composition may be administered by any device to allow the active substance to migrate to the target cell.

The preferred mode of administration and formulation is an injection. The injection may be prepared using aqueous solutions such as physiological saline, Ringer's solution, Hank's solution or sterilized aqueous solution, vegetable oils such as olive oil, higher fatty acid ester such as ethyl oleic acid, and non-aqueous solvents such as ethanol, benzyl alcohol, propylene glycol, polyethylene glycol or glycerin, and the like. For mucosal penetration, a non-penetrating agent known in the art suitable for a barrier to pass through may be used, and may further include a pharmaceutical carrier such as a stabilizer for preventing deterioration such as ascorbic acid, sodium hydrogen sulfite, BHA, tocopherol, EDTA, and the like, an emulsifier, a buffering agent for pH adjustment, and a preservative for inhibiting the growth of microorganisms such as phenylmercuric nitrate, thimerosal, benzalkonium chloride, phenol, cresol, and benzyl alcohol. Methods for formulating pharmaceutical compositions are known in the art, and specific references can be made to literature [Remington's Pharmaceutical Sciences (19th ed., 1995)], etc. The above literature is considered a part of the present specification.

As used herein, the term "pharmaceutically effective amount" refers to an amount sufficient to treat a disease at a reasonable benefit/risk ratio applicable to medical treatment and not causing side effects. The effective dose level can be readily determined by those of ordinary skill in the art based on factors including the patient's sex, age, body weight, health condition, type and severity of the disease, activity of the drug, sensitivity to the drug, method of administration, time of administration, route of administration and excretion rate, duration of treatment, drugs used in combination or simultaneously, and other factors well known in the medical field.

The preferred dosage of the pharmaceutical composition of the present invention may be about $1.0 \times 10^3$ to about $1.0 \times 10^{10}$ cells/kg, about $1.0 \times 10^4$ to about $1.0 \times 10^9$ cells/kg, about $1.0 \times 10^5$ to about $1.0 \times 10^8$ cells/kg, or about $1.0 \times 10^6$ to about $1.0 \times 10^7$ cells/kg per day based on cells, depending on the patient's condition, body weight, sex, and age, severity of the patient, and route of administration.

However, the dosage may be prescribed in various ways depending on factors such as the formulation method, mode of administration, patient's age, body weight, sex, pathological condition, food, administration time, route of administration, excretion rate, and response sensitivity, and those of ordinary skill in the art can appropriately adjust the dosage by considering these factors. The frequency of administration may be once or twice or more within the range of clinically acceptable side effects. Regarding the administration site, it may be administered to 1 site or 2 or more sites. For animals other than humans, it may be administered in the same dosage as humans per kg or per subject, or it may be administered in an amount converted from the above dosage by, for example, the volume ratio (for example, the average value) of the organ (heart, etc.) between the target animal and the human. Such dosage should not be construed as limiting the scope of the present invention in any way.

As used herein, the term "subject" refers to a subject to which the composition of the present invention can be applied (prescribed), and may be a mammal such as a rat, a mouse, or a livestock, including a human. Preferably, it may be a human, but is not limited thereto. In addition to the hypothalamus-like organoid-derived neural stem cells, the pharmaceutical composition of the present invention may further comprise any compound or natural extract that has already been verified as safe and known to have anti-aging activity in order to increase or enhance the hypothalamus-like organoid-derived neural cells or anti-aging activity. At this time, the neural stem cells, the neural cells, or a cell population comprising them; and the compound or natural extract having anti-aging activity may be administered simultaneously or sequentially.

In another aspect of the present invention, there is provided a use of a pharmaceutical composition comprising hypothalamus-like organoid-derived neural stem cells as an active ingredient for the prevention and treatment of geriatric diseases. At this time, the pharmaceutical composition may further comprise hypothalamus-like organoid-derived neural cells. Here, the hypothalamus, the organoid, the neural stem cell, the neural cell, the geriatric disease, the prevention, and the treatment are the same as described above.

In another aspect of the present invention, there is provided a method for preventing and treating geriatric diseases, comprising administering to a subject the pharmaceutical composition comprising hypothalamus-like organoid-derived neural stem cells as an active ingredient. At this time, the pharmaceutical composition may further comprise hypothalamus-like organoid-derived neural cells. Here, the hypothalamus, the organoid, the neural stem cell, the geriatric disease, the administration, the prevention, and the treatment are the same as described above.

The subject may be a mammal such as a rat, a mouse, or a livestock, including a human. Preferably, it may be a human.

The dosage may be about $1.0 \times 10^3$ to about $1.0 \times 10^{10}$ cells/kg, about $1.0 \times 10^4$ to about $1.0 \times 10^9$ cells/kg, about $1.0 \times 10^5$ to about $1.0 \times 10^8$ cells/kg, or about $1.0 \times 10^6$ to about $1.0 \times 10^7$ cells/kg per day based on cells depending on the patient's condition, body weight, sex, and age, severity of the patient, and route of administration. However, the dosage may be prescribed in various ways depending on factors such as the formulation method, mode of administration, patient's age, body weight, sex, pathological condition, food, administration time, route of administration, excretion rate, and response sensitivity, and those of ordinary skill in the art can appropriately adjust the dosage by considering these factors.

**Exosome derived from neural stem cell**

In another aspect of the present invention, there is provided an exosome secreted from a hypothalamus-like organoid-derived neural stem cell. At this time, the hypothalamus, the organoid, and the neural stem cell are the same as described above.

As used herein, the term "exosome" refers to an extracellular vesicle with a size of 50 to 150 nm secreted by cells, also known as a microvesicle. The exosomes are secreted from cells when multivesicular bodies fuse with the cell membrane or are secreted directly through the cell membrane. The exosomes are known to contain a complex mix of factors related to cell differentiation, growth, movement, and signaling, such as intracellular proteins, cell membrane proteins, lipids, and nucleic acids such as RNA, miRNA, and DNA, and thus play an important role in various processes such as coagulation, intercellular signaling, and management of metabolic waste. The diameter of the exosome may be about 30 nm to about 500 nm, about 30 nm to about 400 nm, about 30 nm to about 300 nm, about 30 nm to about 200 nm, about 50 nm to about 200 nm, about 50 nm to about 180 nm, about 75 nm to about 180 nm, or about 50 nm to about 150 nm. The exosome may originate from the plasma membrane or multivesicular bodies (MVBs) and be released or secreted outside the cell.

In the present invention, the exosome may be isolated from a culture of hypothalamus-like organoid-derived neural stem cells, neural cells, or a cell population comprising them.

The hypothalamus-like organoid-derived neural stem cell-derived exosome according to the present invention can prevent or treat geriatric diseases. At this time, the hypothalamus, the organoid, the neural stem cell, the neural cell, the prevention, and the treatment are the same as described above.

The geriatric disease may be a neurodegenerative disease and a muscle aging-related disease. Here, the neurodegenerative disease and the muscle aging-related disease are the same as described above.

**Pharmaceutical composition comprising exosomes derived from neural stem cells**

In another aspect of the present invention, there is provided a pharmaceutical composition for preventing and treating geriatric diseases, comprising the exosomes as an active ingredient. Here, the exosome, the prevention, and the treatment are the same as described above. In addition, the geriatric disease may be a neurodegenerative disease and a muscle aging-related disease, and the neurodegenerative disease and the muscle aging-related disease are the same as described above.

In the pharmaceutical composition for preventing and treating geriatric diseases of the present invention, the exosomes may be included in any amount (effective amount) depending on the purpose, formulation, mixing purpose, and the like, as long as they can exhibit anti-aging activity. The pharmaceutical composition of the present invention may be included in about 1 μg/mL to 1 mg/mL, 10μg/mL to 100 μg/mL, 10 μg/mL to 50 μg/mL, or 10 μg/mL to 20 μg/mL, per 1 mL. Here, the effective amount is the same as described above.

The composition of the present invention may be administered to a subject in a pharmaceutically effective amount. Here, the pharmaceutically effective amount, the administration, and the subject are the same as described above.

The preferred dosage of the pharmaceutical composition comprising the exosomes of the present invention as an active ingredient may be administered at a dosage of 0.001 mg/kg body weight to 100 mg/kg body weight, 0.005 mg/kg body weight to 50 mg/kg body weight, 0.02 mg/kg body weight to 20 mg/kg body weight, or 0.1 mg/kg body weight to 10 mg/kg body weight per day, depending on the patient's condition, body weight, sex, and age, severity of the patient, and route of administration.

The administration may be performed once a day or divided into several times per day. However, the dosage may be prescribed in various ways depending on factors such as the formulation method, mode of administration, patient's age, body weight, sex, pathological condition, food, administration time, route of administration, excretion rate, and response sensitivity, and those of ordinary skill in the art can appropriately adjust the dosage by considering these factors. The frequency of administration may be once or twice or more within the range of clinically acceptable side effects. Regarding the administration site, it may be administered to 1 site or 2 or more sites. For animals other than humans, it may be administered in the same dosage as humans per kg or per subject, or it may be administered in an amount converted from the above dosage by, for example, the volume ratio (for example, the average value) of the organ (heart, etc.) between the target animal and the human. Such dosage should not be construed as limiting the scope of the present invention in any way.

In some cases, it may be considered that the pharmaceutical composition of the present invention is loaded into a vehicle comprising a means for directing exosomes to a lesion. Therefore, the pharmaceutical composition of the present invention may be administered through several routes including topical (including buccal, sublingual, dermal and intraocular administration), parenteral (including subcutaneous, intradermal, intramuscular, instillation, intravenous, intraarterial, intraarticular and intracerebrospinal fluid) or transdermal administration, and preferably, may be administered directly to the site of disease. However, the administration method is not limited thereto, and a method suitable for treatment can be used.

The preferred mode of administration and formulation of the pharmaceutical composition of the present invention is an injection. Methods for formulating pharmaceutical compositions are known in the art, and specific references can be made to literature [Remington's Pharmaceutical Sciences (19th ed., 1995)], etc. The above literature is considered a part of the present specification.

In addition, the composition of the present invention may comprise a pharmaceutically acceptable carrier. The composition and the pharmaceutically acceptable carrier are the same as described above. The pharmaceutically acceptable carrier means an excipient, a diluent, or an adjuvant. Examples of carriers may be selected from the group consisting of lactose, dextrose, sucrose, sorbitol, mannitol, xylitol, erythritol, maltitol, starch, acacia gum, alginate, gelatin, calcium phosphate, calcium silicate, cellulose, methylcellulose, polyvinylpyrrolidone, physiological saline, buffers such as PBS, methylhydroxybenzoate, propylhydroxybenzoate, talc, magnesium stearate, and mineral oil. The composition may comprise a filler, an anticoagulant, a lubricant, a wetting agent, a flavoring agent, an emulsifier, a preservative, and the like.

In addition to the exosome as an active ingredient, the anti-aging composition of the present invention may further comprise a cell therapeutic agent (for example, a neural stem cell, a neural cell, or a cell population comprising them), any compound or natural extract that has already been verified as safe and known to have anti-aging activity in order to increase or enhance anti-aging activity. At this time, the exosome and the cell therapeutic agent, compound, or natural extract having anti-aging activity may be administered simultaneously or sequentially.

In another aspect of the present invention, there is provided a use of an exosome isolated from a hypothalamus-like organoid-derived neural stem cell for the prevention and treatment of geriatric diseases. Here, the hypothalamus, the organoid, the neural stem cell, the neural cell, the exosome, the geriatric disease, the prevention, and the treatment are the same as described above.

In another aspect of the present invention, there is provided a method for preventing and treating geriatric diseases, comprising administering to a subject the exosome. Here, the exosome, the subject, the administration, the geriatric disease, the prevention, and the treatment are the same as described above.

The preferred dosage of the exosome may be administered at a dosage of 0.001 mg/kg body weight to 100 mg/kg body weight, 0.005 mg/kg body weight to 50 mg/kg body weight, 0.02 mg/kg body weight to 20 mg/kg body weight, or 0.1 mg/kg body weight to 10 mg/kg body weight per day, depending on the patient's condition, body weight, sex, and age, severity of the patient, and route of administration.

The administration may be performed once a day or divided into several times per day. However, the dosage may be prescribed in various ways depending on factors such as the formulation method, mode of administration, patient's age, body weight, sex, pathological condition, food, administration time, route of administration, excretion rate, and response sensitivity, and those of ordinary skill in the art can appropriately adjust the dosage by considering these factors.

**Method for producing exosomes derived from neural stem cells**

In another aspect of the present invention, there is provided a method for producing an exosome, comprising the steps of: culturing hypothalamus-like organoid-derived neural stem cells and collecting exosomes from the culture fluid. Here, the hypothalamus, the organoid, the neural stem cell, and the exosome are the same as described above.

**Kit comprising exosomes derived from neural stem cells and/or neural stem cells**

In another aspect of the present invention, there is provided a kit for preventing and treating geriatric diseases, comprising hypothalamus-like organoid-derived neural stem cells; and/or exosomes secreted from hypothalamus-like organoid-derived neural stem cells. At this time, the hypothalamus, the organoid, the neural stem cell, the exosome, the prevention, and the treatment are the same as described above.

The geriatric disease may be a neurodegenerative disease, osteoporosis, and a muscle aging-related disease. Here, the neurodegenerative disease and the muscle aging-related disease are the same as described above.

**Mode for Carrying out the Invention**

Hereinafter, the present invention will be described in more detail by way of the following examples. However, the following examples are only for illustrating the present invention, and the scope of the present invention is not limited only to these examples.

**Experimental Example 1. Cell culture**

Human embryonic stem cell line H9 (H9 hESCs, WiCell) was cultured on a matrigel-coated plate in mTeSR-1 medium (Stem Cell Technology) in the absence of feeder cells. Human hypothalamus-derived neural stem cells (ntNSCs) were produced from H9 hESCs using a 3D culture method.

Specifically, hESCs were suspended in neural induction medium at a density of 10,000 cells per well in a low-adhesion 96-well round-bottom plate, inoculated, and cultured for 24 hours. At this time, the neural induction medium used was a 1:1 mixed medium of Neurobasal medium (Gibco) and N2 medium containing B27 without vitamin A (Invitrogen) and 200 $\mu$M ascorbic acid (Sigma) and 1.25 mg/L insulin, supplemented with 10 $\mu$M SB431542 (Tocris), 200 ng/mL noggin (Peprotech), and 20 $\mu$M Y27632 (ROCK inhibitor, Sigma).

For hypothalamus patterning of H9 hESCs, they were cultured for 5 days in neural induction medium containing 10 $\mu$M SB431542 (Tocris) and 200 ng/mL noggin (Peprotech), and then cultured for 6 days by adding 100 ng/mL sonic hedgehog (SHH, Peprotech) and 2 $\mu$M purmorphamine (Calbiochem) to the medium. On the 12th day after the start of differentiation, the formed spheres were transferred to a low-adhesion 6-well plate and cultured in neural expansion medium for 8 days (until the 20th day after the start of differentiation) using an orbital shaker (80 rpm). The neural expansion medium used was N2 medium containing 1.25 mg/L insulin, 200 $\mu$M ascorbic acid, 20 ng/L bFGF (Peprotech), and penicillin/streptomycin. Thereafter, the spheres were chopped and suspended in neural expansion medium and inoculated into a poly-L-ornithine/fibronectin-coated plate. The neural stem cells obtained through the above process were cultured in N2 medium (neuron differentiation medium) containing 1.25 mg/L insulin, 200 $\mu$M ascorbic acid, 10 ng/mL BDNF (Peprotech), 10 ng/mL GDNF (Peprotech), 125 $\mu$M db-cAMP (Sigma), and penicillin/streptomycin, and differentiated into neural cells.

**Experimental Example 2. Animal experiment**

All procedures for animal experiments were conducted with the approval from the Institutional Animal Care and Use Committee (IACUC) of Hanyang University College of Medicine (Approval Nos. 2018-0217A and 2020-0142A) and the IACUC and Institutional Biosafety Committee (IBC) of the Korea Institute of Science and Technology (KIST) (Approval No. KIST-2019-048). For animal experiments, 11-month-old middle-aged C57BL/6 male mice were purchased from the Korea Basic Science Institute or Janvier Lab. The mice were raised in a breeding room with constant temperature (23$\pm$1 °C), constant humidity (50$\pm$10 %), and a 12-hour light/dark cycle, with free access to food and water.

**Experimental Example 3. Cell transplantation experiment**

For cell transplantation, 12-month-old male C57BL/6J mice were used. First, the mice were anesthetized with zoletil (37.5 mg/kg) and rompun (5.83 mg/kg), and then human hypothalamus-derived NSCs (1 μL, 2 × 10⁵ cells/uL) suspended in N2 medium were injected into the mediobasal hypothalamus (MBH) at a rate of 250 nL/min for 2 minutes (AP: -1.5 mm, ML: ±0.4 mm, DV: -5.6 mm). After the injection, the injector was left in place for an additional 5 minutes without being removed.

### Experimental Example 4. Histological analysis

The mouse was anesthetized and perfused intracardially with 30 mL PBS (pH 7.4) to remove blood, and then perfused intracardially with 4% paraformaldehyde (PFA, 30 mL) diluted in PBS to fix the tissue. The fixed brain tissue was extracted and further fixed overnight with 4% PFA (diluted in PBS) at 4 °C. The fixed brain tissue was reacted with 30% sucrose for 7 days to remove moisture in the tissue (cryoprotection). The dehydrated brain tissue was molded with TissueTek O.C.T compound (Sakura Finetek), frozen, and then sectioned at a thickness of 30 μm using a cryostat (Leica) to prepare brain tissue sections.

For peripheral tissues, white adipose tissue (WAT), brown adipose tissue (BAT), liver, and femur, which were extracted after dissection, were separated and stored for protein analysis and immunostaining. For tissue staining, each tissue was fixed overnight at 4 °C in 4% formaldehyde (PFA), then embedded in paraffin, and sectioned at a thickness of 4 μm to prepare tissue sections. Tissue staining was performed in the same manner as the known H&E method.

### Experimental Example 5. Immunostaining

The cells and cryo-sectioned brain tissue sections were fixed in 4% paraformaldehyde (PFA) diluted in PBS, then treated with 0.3% Triton X-100 containing 1% BSA, and reacted for 1 hour. Thereafter, they were treated with anti-Nestin antibody (1:500, BioLegend, 65680), anti-Rax antibody (1:500, Takara, M228), anti-Sox2 antibody (1:200, ThermoFisher, MA5-13961), anti-Nkx2-1 antibody (1:10, DSHB, 40.2D6), anti-KI67 antibody (1:500, Abcam, ab15580), anti-TUJ1 antibody (1:200, BioLegend, 801202), anti-MAP2 antibody (1:200, Sigma-Aldrich, M1406), anti-RBFOX3 antibody (1:200, Millipore, MAB377), anti-SYN1 antibody (1:200, Millipore, AB1543), anti-αMSH antibody (1:2000, Phoenix Pharmaceuticals, H-043-01), anti-NPY antibody (1:1000, ImmunoStar, 22940), anti-SCGN antibody (1:500, Sigma-Aldrich, HPA006641), anti-PMCH antibody (1:500, Sigma-Aldrich, M8440), anti-GFAP antibody (1:500, Agilent, Z0334), anti-hNCAM antibody (1:500, Santa Cruz Biotechnology, sc-106), anti-hGFAP antibody (1:400, BioLegend, 837201), or anti-huMt antibody (1:500, Abcam, ab92824) as primary antibodies, and reacted overnight at 4 °C in a light-protected environment. For visualization, anti-mouse IgG (1:1000, Jackson Immunoresearch Laboratories, 111-165-003 or A-11001) conjugated with Cy3 or Alex Flour 488 was used as a secondary antibody.

The stained cells and brain tissue sections were mounted using VECTASHIELD mounting solution (Vector Laboratories) containing DAPI and observed using a fluorescence microscope (Leica).

### Experimental Example 6. Quantitative RT-PCR analysis

RNA was extracted using TRIzol™ reagent (Invitrogen) according to the manufacturer's protocol, and then cDNA was synthesized using the SuperScript™ kit (Invitrogen). Quantitative RT-PCR (q-PCR) was performed on a CFX96™ Real-Time System using iQTM SYBR® Green Supermix (Bio-Rad), and gene expression levels were quantified as GAPDH or beta-actin expression levels. At this time, the primers used are shown in Table 1.

[Table 1]

| Gene | Forward | SE Q ID NO | Reverse | SE Q ID NO |
|---|---|---|---|---|
| NKX2-1 | CAGGACACCATGAGGAACAGCG | 1 | GCCATGTTCTTGCTCACGTCCC | 2 |
| RAX | GCAAGGTCAACCTACCAGAGGT | 3 | GCAGCTTCATGGAGGACACTTC | 4 |
| OTP | AGCTTCGCCAAGACTCACTACC | 5 | CACGGAACACGTTGGTCGTCTT | 6 |
| ISL1 | GCAGAGTGACATAGATCAGCCTG | 7 | GCCTCAATAGGACTGGCTACCA | 8 |
| SF-1 | CCAGACCTTCATCTCCATCGTG | 9 | TGGCGGTAGATGTGGTCGAACA | 10 |
| EN1 | GCAACCCGGCTATCCTACTTATG | 11 | ATGTAGCGGTTTGCCTGGAAC | 12 |

(continued)

| Gene | Forward | SEQ ID NO | Reverse | SEQ ID NO |
|------|---------|-----------|---------|-----------|
| *ACTB* | GTGACAGCAGTCGGTTGGAG | 13 | AACAACGCATCTCATATTTGGA A | 14 |

### Experimental Example 7. Western blot

RIPA buffer (50 mM Tris-HCl pH 7.4, 150 mM NaCl, 1% Triton X-100, 1% sodium deoxycholate, 0.1% SDS, and 1 mM EDTA) containing protease inhibitors and phosphorylation enzyme inhibitors was added to the cells and sonicated to lyse the cells. The cell lysate was centrifuged at 4 °C and 15,000g for 5 minutes to obtain the supernatant, which was then quantified using Pierce BCA analysis (Thermo Fisher). The proteins separated by SDS-PAGE electrophoresis were transferred to a nitrocellulose membrane, and then TBST containing 5% BSA was added, and reacted for 1 hour for blocking. After blocking, they were treated with the primary antibodies as shown in Table 2 and reacted overnight at 4 °C. After the primary antibody reaction, goat-anti-rabbit-HRP conjugate or goat-anti-mouse-HRP conjugate (Cell Signaling Technology) as the secondary antibody was diluted 1: 1,000 in TBST containing 5% BSA, treated on the membrane, and reacted for 1 hour. The protein expression for each antibody was confirmed using West-Pico chemiluminescent substrate (Thermo Fisher). Leptin and ghrelin activity analysis was performed by treating differentiation-induced cells with leptin (100 ng/mL) or ghrelin (10 nM), respectively, and then reacting for 1 hour.

[Table 2]

| Antibody | Dilution ratio | Supplier | Product number |
|----------|----------------|----------|----------------|
| Rabbit polyclonal anti-phospho-STAT3(Tyr705) | 1:1000 | Cell Signaling Technology | Cat# 9131 |
| Rabbit polyclonal anti-STAT3 | 1:1000 | Cell Signaling Technology | Cat# 9132 |
| Rabbit polyclonal anti-phospho-FoxO1(Ser256) | 1:1000 | Cell Signaling Technology | Cat# 9461 |
| Rabbit polyclonal anti-FoxO1(C29H4) | 1:1000 | Cell Signaling Technology | Cat# 2880 |
| Mouse monoclonal anti-GAPDH(0411) | 1:1000 | Santa Cruz Biotechnology | Cat# sc-47724 |
| Mouse monoclonal anti-MTCO1(1D6E1A8) | 1:1000 | Thermo Fisher Scientific | Cat# 459600 |
| Mouse monoclonal anti-SDHB(G-10) | 1:1000 | Santa Cruz Biotechnology | Cat# sc-271548 |
| Rabbit polyclonal anti-FGF21 | 1:1000 | Abcam | Cat# ab137715 |
| Rabbit polyclonal anti-Beta Actin | 1:1000 | Proteintech | Cat# 20536-1-AP |
| Rabbit monoclonal anti-phospho-eIF2alpha(Ser51) (119A11) | 1:1000 | Cell Signaling Technology | Cat# 3597 |
| Rabbit monoclonal anti-eIF2alpha(D7D3) | 1:1000 | Cell Signaling Technology | Cat# 5324 |
| Rabbit monoclonal anti-ATF-4(D4B8) | 1:1000 | Cell Signaling Technology | Cat# 11815 |
| Goat polyclonal anti-GDF15 | 1:1000 | Abcam | Cat# ab39999 |

### Experimental Example 8. Exosome isolation and mouse treatment

The exosomes in the cell culture medium were purified using an ultracentrifuge. Specifically, the cell culture fluid was centrifuged to obtain the supernatant, and then centrifuged at 300 g for 10 minutes, at 2000 g for 10 minutes, at 10,000 g for 30 minutes, and at 100,000 g for 1 hour in an ultracentrifuge, filtered, and then centrifuged again at 100,000 g for 1 hour in an ultracentrifuge to obtain the exosomes.

The exosomes isolated through the above process were finally resuspended in PBS and prepared.

For all mice, a 26-gauge guide cannula was transplanted at the midline coordinates of 2.0 mm posterior to the bregma and 5.0 mm inferior to the bregma in the third ventricle (3V) of the hypothalamus using an ultra-precision small animal stereotaxic device (Kopf Instruments, Tujunga, CA, USA) in the same manner as in Experimental Example 2. Among these, the vehicle (PBS) and exosome-treated groups were injected with PBS or exosomes through the cannula into the 3V three times a week for three months.

## Experimental Example 9. Mouse behavior experiment

All behavior experiments were performed in a behavior laboratory, and all behavior evaluations were recorded using a computerized ANY-maze video-tracking system (Stoelting). In addition, all experiments were performed according to the ARRIVE guidelines.

### Experimental Example 9.1. Muscle strength evaluation (grip strength test)

The mouse was placed on a square grid (1 cm mesh size), and then the grid was turned over, and the time the mouse spent hanging with its feet was measured and recorded for 2 minutes. The experiment was repeated three times with at least 10 minutes of rest for each mouse.

### Experimental Example 9.2. Motor coordination evaluation (rotarod test)

The mice were trained for 60 seconds on a rotarod (B.S Technolab INC.) moving at a speed of 5 rpm, and then rested for 10 minutes. Then, each mouse was placed on a rotarod moving at a speed of 5 rpm, and the mouse's behavior was recorded while increasing the speed by 5 rpm per minute for 5 minutes. The experiment was repeated three times with 30 minutes of rest for each mouse.

### Experimental Example 9.3. Endurance evaluation (treadmill test)

Endurance evaluation was performed using a treadmill (Jeung Do Bio & Plant Co., Ltd.) that can evaluate up to 8 mice in an individual lane. Before conducting the experiment, the mice were acclimatized to the treadmill at a speed of 5 rpm for 5 minutes to prevent running injuries and failures. Thereafter, the speed was increased by 2 rpm per minute for 10 minutes, and the time until the mice gave up running was recorded.

### Experimental Example 9.4. Novel object recognition test

First, before conducting the experiment, the mice were placed freely in an open field box (40 cm in width $\times$ 40 cm in length $\times$ 50 cm in height) for 10 minutes. Then, the mice were allowed to freely recognize two objects of the same shape (first session). Thereafter, one of the two objects was replaced with a novel object, and then the mice were exposed for 10 minutes (second session). Then, the time the mouse spent recognizing each object was recorded. The preference index was expressed as the ratio of the time spent recognizing one of the two objects (including the novel object) to the total time spent recognizing the two objects.

### Experimental Example 9.5. Morris water maze test

The Morris water maze test was conducted in the following manner.
First, a water tank was filled with water at 24 °C, and the background of the water tank was made opaque white by painting it with Crayola non-toxic paint. Then, the water tank was placed in the center of a room with an extra-maze cue (various black shapes on a white background). At this time, the water tank was divided into four quadrants (northwest, northeast, southwest, and southeast) with a diameter of 90 cm. Then, a circular platform with a diameter of 10 cm was placed 25 cm away from the tank wall, and the time, distance, and speed taken by the mouse to move from the same starting position to the platform for each experiment were measured.
The experiment was conducted in two stages: hidden platform training and probe trial. Hidden platform training was conducted as follows. The mouse was first trained to swim to a visible platform installed 0.5 cm above the water surface and stay there for 10 seconds. At this time, if the mouse did not find the platform within 60 seconds, a glass stirring rod was used to guide it to the platform. The training was conducted four times a day for four consecutive days with different access locations (access locations were north, south, east, and west). After the training was conducted, the platform was installed 1 cm deep under the water surface so that it was not visible. Thereafter, the delayed time until the mouse reached the platform, the distance moved, the path efficiency, the time spent in each quadrant and the distance moved, the total distance moved, and the swimming speed were measured. For the probe trial, on the fifth day after the start of training, the platform was removed and the trained mouse was started at a location opposite to where the platform had been installed (target quadrant). The time spent by the mice in all quadrants for 60 seconds, the distance and number of times they crossed the target quadrant, the total distance moved, and the swimming speed were recorded. Learning and memory of the mouse were evaluated by the total time spent in the target quadrant during the probe trial.

**Experimental Example 9.6. Y maze test**

The measuring equipment used a Y maze consisting of three identical branches (40 cm in length - 4 cm in width - 15 cm in height) with each branch folding at a 120° angle. The three branches were designated as A, B, and C, respectively, and the mouse was placed at the end of one branch and allowed to move to the other branch for 10 minutes. The number of times the experimental animal entered the branch up to its tail and the number of times it entered each branch in sequence were counted and 1 point (actual alternation) was awarded. Alternation behavior is defined as non-overlapping occurrence of all three (e.g., ABC, BCA, CAB, but not ABA), and spontaneous alternation was calculated using the following mathematical equation 1.

<Mathematical equation 1>

Spontaneous alteration = [(number of alternations) / (total items - 2)] × 100

**Experimental Example 9.7. Open field test**

The mice were placed one by one in the center of the open field box (40 cm in length $\times$ 40 cm in width $\times$ 50 cm in height) and acclimatized for 20 minutes. The movements of the mice were recorded for 10 minutes and then analyzed using a digital camera connected to the Any-Maze animal tracking system software (Stoeling, Wood Dale). At this time, the total distance moved (meter) and the time spent in the center and periphery of the open field box (second) were measured.

**Experimental Example 9.8. Passive avoidance test**

There was a gate in the center of the measuring device (48 cm in length $\times$ 23 cm in width $\times$ 28 cm in height), and the area within the measuring device was divided equally into a lighted compartment (bright compartment) and a dark compartment. On the first day, the mouse was allowed to freely recognize the two compartments for 10 minutes. On the next day (training), the mouse was left in the lighted compartment for 60 seconds, and then the next gate was opened so that the mouse could freely enter the dark compartment. When the mouse entered the dark compartment, the door was closed and an electric shock (0.3 mA, 3 seconds) was applied to the mouse's foot. At this time, the latency for each stage or the time it took for the mouse to enter the dark compartment was recorded. On the third day (probe trial), the mouse was placed in the lighted compartment, and after 60 seconds, the gate was opened, and the time until the mouse moved to the dark area inside the gate was measured. The latency for each stage was measured for 540 seconds.

**Experimental Example 10. Body composition analysis**

As in Experimental Example 3, the fat and lean percentage of the cell-transplanted mice were measured using minispec LF50 (Bruker) at the Korea Mouse Phenotyping Center (KMPC).

**Experimental Example 11. Glucose tolerance test**

As in Experimental Example 3, the cell-transplanted mice were fasted for 12 hours, and then glucose was administered intraperitoneally at a concentration of 2 g/kg. After glucose administration, blood was collected from the tail at 0, 30, 60, and 120 minutes, and blood glucose levels were measured using a glucometer.

**Experimental Example 12. Metabolic cage and behavior analysis**

In order to measure metabolic rate, the metabolic parameters of mice were analyzed by indirect calorimetry 1-2 using a CLAMS (comprehensive laboratory animal monitoring system) system (Columbus Instruments, Columbus, OH, USA) at room temperature (22 °C). Two to three days before the experiment, the mice were housed in a single measurement cage (20.5 cm in length $\times$ 10.5 cm in width $\times$ 12.5 cm in height) and fed the same diet (normal diet or high-fat diet) to acclimatize them. $O_2$ consumption, $CO_2$ production, energy expenditure, heat, and locomotor activity of the experimental animals were measured for 24 hours. The above measurement data were expressed by calculating the respiratory quotient or $CO_2/O_2$ exchange ratio and $\Delta$heat value using CLAX software (version 2.2.15, Columbus Instruments).

**Experimental Example 13. Bulk RNA-sequencing transcriptome analysis**

Total RNA (RIN>8) was isolated using TRIzol (approximately $2\times10^6$ cells), and a cDNA library was prepared using TruSeq Stranded mRNA LT (Illumina). Sequencing was performed using NovaSeq 6000 (Macrogen). The paired-end library was analyzed by comparing (aligning) with the human reference UCSC hg19 using the HISAT2 pipeline. The DESeq2 method was used to analyze differentially expressed genes (DEGs) showing differences in expression levels between the control and sample groups. At this time, genotype-tissue expression (GTEx) data were used by randomly selecting four samples each from the hypothalamus, hippocampus, cortex, and substantia nigra of postmortem donors from dbGaP (The database of Genotypes and Phenotypes) (study accession phs000424.v7. p2; https://www.ncbi.nlm. nih.gov/projects/gap/cgi-bin/study.cgi?study_id=phs000424.v7.p2).

**Experimental Example 14. Single cell RNA nucleotide sequence analysis**

htNSCs were cultured in a neurosphere state in neuron differentiation medium for 70 days.

The cultured hypothalamus-like organoids were treated with papain solution (PAP2, Worthington) and DNAse solution (D2, Worthington) at a ratio of 20:1, reacted at 37 °C for 20 minutes, and centrifuged at 500g for 5 minutes to separate cells. The cell pellet obtained by the above method was resuspended in neuron differentiation medium containing 0.004% BSA, and cell viability was measured. A single cell cDNA library was produced for the separated cells using the Chromium Next GEM Single Cell 3' RNA library v3.1 Reagent Kit (10X Genomics, USA). The single cell cDNA library was sequenced with the NextSeq 500 system (Illunimia) to obtain 91 bp and 28 bp paired end reads. The above results were de-multiplexed and quantified based on the UMI (Unique Molecule Identifier) using Cell Ranger version 5.0.0 (10X Genomics, USA).

The count matrix was generated for each sample with default parameters, and the genes were mapped based on the human reference genome GRCh38.

**Experimental Example 15. Cell proliferation analysis test**

Cell proliferation was confirmed through the population doubling level (PDL) calculated by the mathematical equation 2.

$$<\text{Mathematical equation 2}>$$
$$\text{Population doubling level (PDL)} = \log (N/N_0) \, / \log 2$$

At this time, N refers to the number of cells at the end of each passage, and $N_0$ refers to the number of cells inoculated at the start of the culture.

**Experimental Example 16. Cell viability analysis**

Cell viability was measured using the LIVE/DEAD Viability/Cytotoxicity Kit (ThermoFisher #L3224).

**Experimental Example 17. Bone micro-CT analysis test**

The right femur of each mouse was extracted and fixed overnight in 4% formaldehyde. The fixed femurs were placed in a scanning tube with a diameter of 12.3 mm, and images were taken using a desktop micro-CT ($\mu$CT) scanner (SkyScan 1076, Bruker), and the scanned images were merged into a 3D image. The total tissue volume, bone mineral density (BMD), bone volume (BV)/tissue volume (TV), trabecular number (Tb. N), and trabecular thickness (Tb. Th) were analyzed in 100 slides taken from approximately 0.5 mm to 1.6 mm from the growth plate as the region of interest (ROI).

**Experimental Example 18. Statistical analysis**

Statistical analysis was performed using Prism software version 6 (GraphPad Software) or IBM SPSS Statistics 26 (IBM) using a two-tailed unpaired sample t-test, one-way analysis of variance (ANOVA), or Mann-Whitney U test. All results were considered statistically significant when $p < 0.05$. Error bars are expressed as mean $\pm$ standard error (SEM) or median, and p values are expressed as *$p < 0.05$, **$p < 0.01$, ***$p < 0.001$, ****$p < 0.0001$.

**Experimental Result 1. Production of htNSCs from hPSC-derived hypothalamus-like organoids**

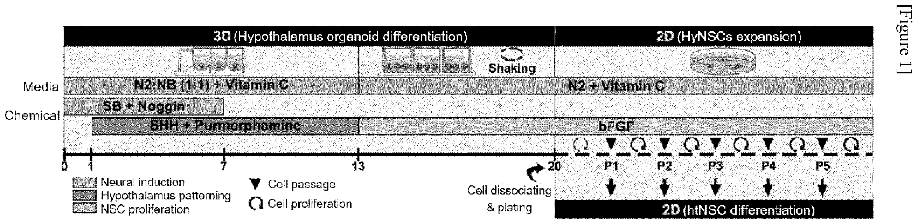

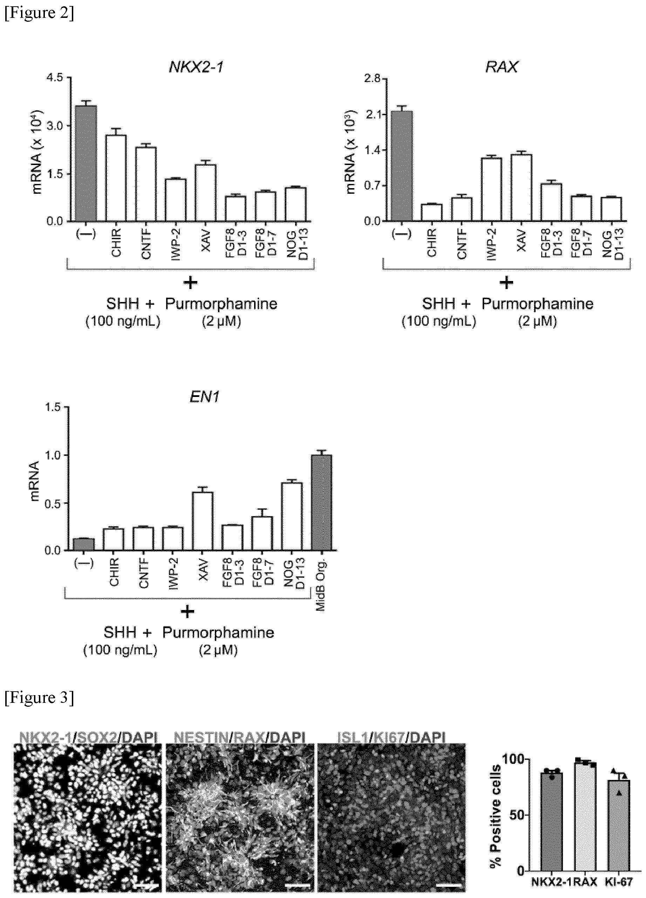

In order to provide a stable cell source for human hypothalamic neural cells and non-neural cells for a cell therapeutic agent, a protocol for differentiating hPSCs into hypothalamic NSCs (htNSCs) was developed. hESCs (H9) were cultured in 3D, and differentiation from hESCs into NSCs was induced through orbital shaking in the presence of a substance that induces patterning into the neuroepithelial and hypothalamic regions (Figure 1). The concentration and treatment time of the hypothalamus patterning-inducing substance were optimized based on the expression of RAX, NKX2-1, and ILS1, which are known to be highly expressed in the hypothalamus, and Engrailed-1 (EN1), which is known to be lowly expressed (Figure 2). Specifically, it was confirmed that when treated with sonic hedgehog (SHH) and purmophamine simultaneously, the expression of hypothalamus neural stem cell markers NKX2.1 and RAX was increased, while the expression of midbrain marker Engrailed-1 (EN1) was decreased. In addition, treatment with other small molecules known to be associated with hypothalamus patterning (CHR, CNTF, IWP-2, XAV, FGF8, NOG) reduced the expression of hypothalamus markers and simultaneously increased the expression of the midbrain marker EN1. Therefore, in the present invention, hypothalamus patterning was performed using only SHH and purmophamine.

The organoids patterned under the above conditions showed gene expression similar to the hypothalamus differentiation pattern on day 20 of culture.

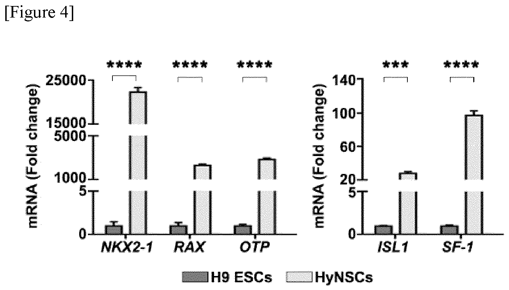

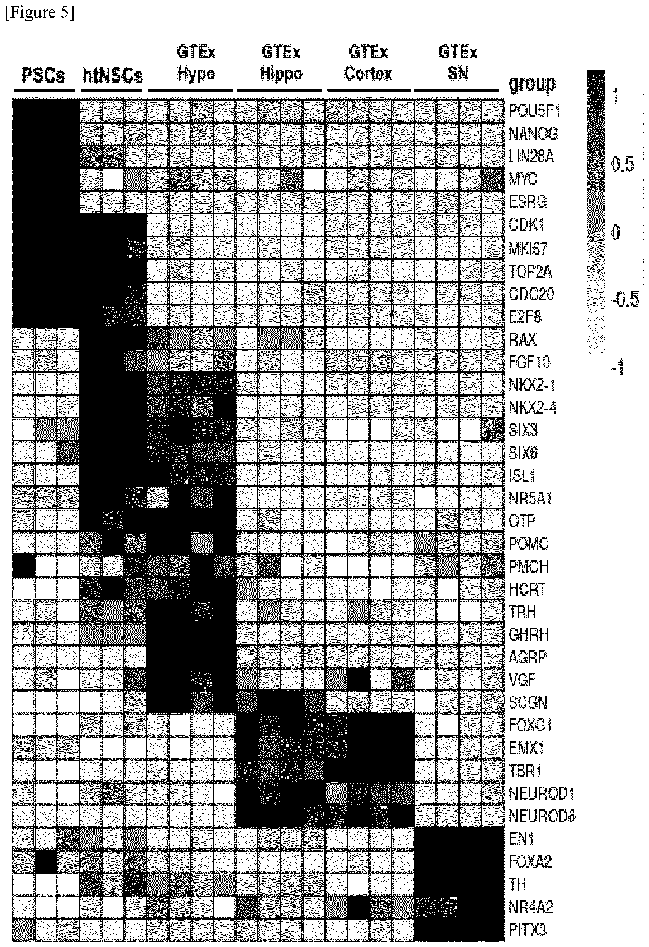

In particular, fibroblast growth factor (bFGF) increased htNSCs in the early hypothalamus-like organoids. On day 20 (D20) of culture, the cells were isolated from the organoids and subcultured using N2 medium containing bFGF on a fibronectin-coated plate. As a result, the expression of NSC markers (SOX2, NESTIN, KI67) and hypothalamus region-specific markers (RAX, NKX2-1, ISL1, OTP, SF-1) was observed in 84-97% of the cultured cells (Figures 3 and 4). As a result of comparative analysis of the transcript of the differentiated htNSC and the transcript of human fetal brain (GTEX) by region, it was confirmed that the differentiated htNSC transcript was clustered with the human fetal hypothalamus and had characteristics similar to the hypothalamus region (Figure 5).

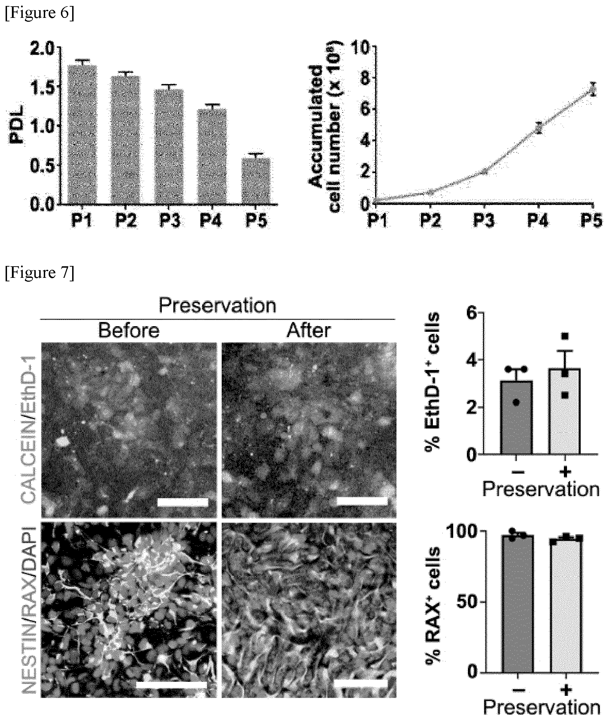

In addition, the htNSC maintained cell growth even after multiple passages (approximately 5 passages) (Figure 6). The htNSCs were cryopreserved and then re-cultured, and the expression of cell death (ethidium heterodimer-1, EthD-1), cell survival (calcein), and neural stem cell (Rax, nestin) marker proteins was confirmed. As shown in Figure 7, it was confirmed that most of the thawed cells survived, and the expression level of hypothalamus neural stem cell marker proteins was similar to that of the cells before freezing. Based on the above results, it was confirmed that the htNSCs can be cryopreserved and maintain the characteristics of htNSCs even after thawing.

**Experimental Result 2. Analysis of differentiation phenotype of organoid-derived human htNSCs**

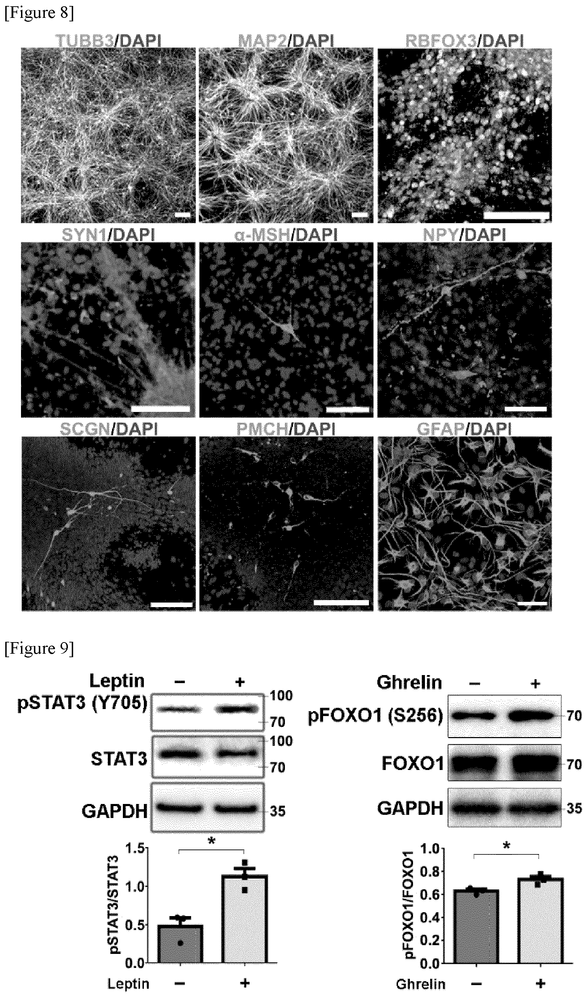

Differentiation from htNSCs into neural cells was induced. The induced neural cells expressed neuron markers (synapsin1 (SYN1), TuJ1 (TUBB), MAP2, RBFOX3 (NeuN)), and a subset of cells expressing hypothalamus specific neuropeptides ($\alpha$-MSH, Neuropeptide Y (NPY), secretagogin (SCGN), PMCH) was also observed (Figure 8).

In particular, GFAP$^+$ astrocytes were also observed in differentiated htNSC cultures, and it was confirmed that glial cells increased at the end of differentiation. In addition, the expression of leptin receptors and ghrelin receptors was observed in cells differentiated from htNSCs (Figure 8), and it was confirmed that when the cells were treated with leptin or ghrelin, the phosphorylation of STAT3 or FOXO1, which are downstream signaling factors of each receptor, was increased (Figure 9).

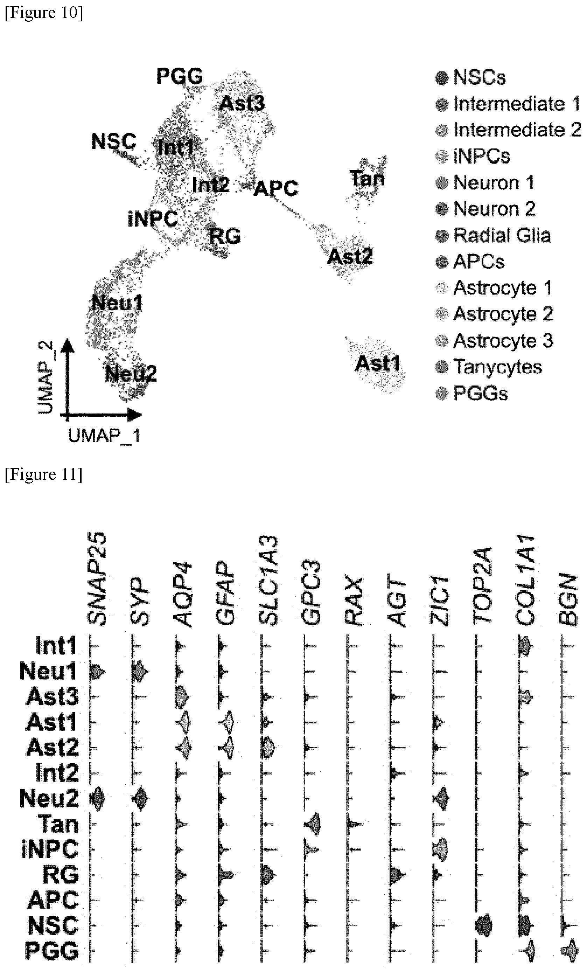

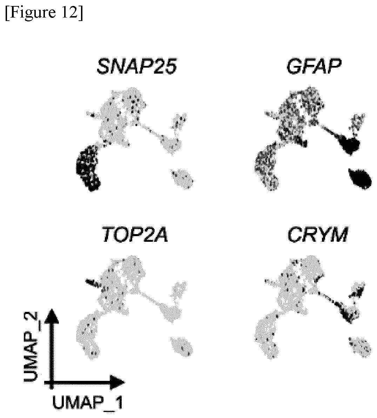

More specifically, in order to analyze the phenotype of cells differentiated from htNSCs, RNA sequencing (sc-RNA-Seq) analysis was performed on day 70 of differentiation. A total of 11,813 cells were sequenced using the Smart-seq2 method, and the read values were filtered to remove doublets and unreliable values, and a total of 6,394 transcripts were analyzed. First, the results of clustering using the Seurat algorithm were divided into 13 clusters, which were visualized by a uniform manifold approximation (UMAP) map. Based on the expression levels of well-known marker genes within the above clusters (Artegiani et al., 2017; Hochgerner et al., 2018; Zeisel et al., 2018), the clusters were divided into neural stem/progenitor cells (NSCs), intermediate progenitor cells (Int1 and Int2), astrocyte progenitor cells (APCs), astrocytes (Ast1, Ast2, and Ast3), tanycytes (Tan), interneuron progenitor cells (Int1 and Int2), radial glia (RG), and proteoglycan-secreting glia (Figure 10). At this time, it was confirmed that specific genes were expressed by each cluster (Figures 11 and 12).

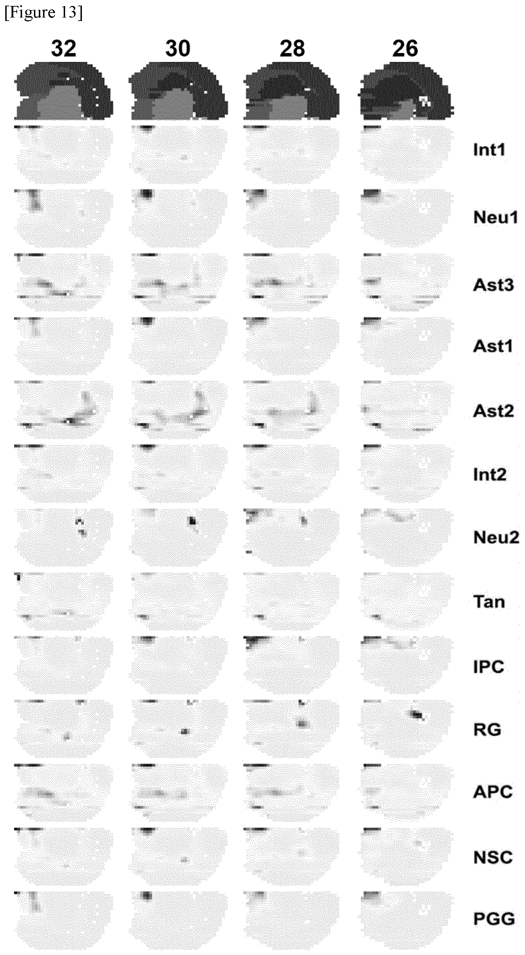

In order to reconfirm the above analysis results, Voxhunt was used to compare and analyze the mouse brain transcript (14 days old) of the Allen developing brain atlas. As a result of the analysis based on voxel-based spatial resolution, it was confirmed that all cell clusters differentiated from htNSCs were precisely located in the hypothalamus of the mouse brain at 14 days old. In addition, it was confirmed that the progenitor/intermediate cell cluster (Int1, Int2, IPC, APC, and NSC) was concentrated in the hypothalamus region adjacent to the ventricular region where adult neuron differentiation occurs (Figure 13). Based on the above results, it was confirmed that the neural stem cells by the method of the present invention are hypothalamus-specific.

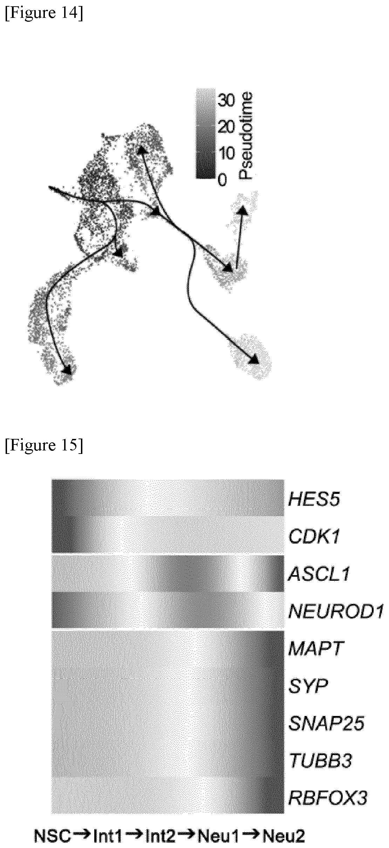

In addition, similar to the differentiation of neural cells and astrocytes from the same progenitor cells, the UMAP clustering results confirmed that the intermediate progenitor cell cluster (Int1 and Int2) was bi-differentiated into the neural cell cluster (Neu1 and Neu2) and the astrocyte progenitor cell cluster (APC) (Figure 14). When the results of analyzing the progenitor/intermediate cell cluster (Int1, Int2, IPC, APC, and NSC) using the pseudo-temporal ordering method using Monocle3 were examined, it was confirmed that neural stem cells were differentiated in the order of the intermediate cell cluster (Int1, Int2) and neural cells (Neu1, Neu2). In addition, it was confirmed that as differentiation progressed, the expression of the cell proliferation marker CDK1 was rapidly reduced in the early progenitor cell stage, the expression of the progenitor neural cell gene ASCL1 was increased in the late progenitor cell stage, and the expression of the neural cell-specific genes TUBB3 and SNAP25 was increased in the late neural cell stage (Figure 15).

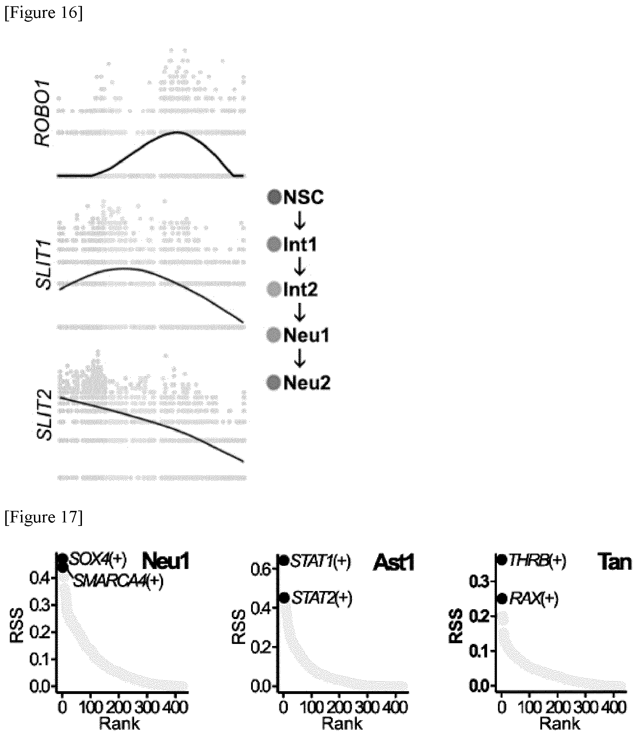

SLIT-ROBO signaling has recently been reported as an important regulator of hypothalamus-specific brain development (Romanov et al. 2020). In the cells induced to differentiate from the htNSCs of the present invention, a SLIT-ROBO signaling gene expression pattern similar to the *in vivo* hypothalamus brain development was confirmed (Figure 16). Based on the above results, it was confirmed that the differentiation pattern of the organoid-derived human htNSCs was similar to *in vivo* hypothalamus development.

Next, a gene regulatory network (GRN) analysis was performed using pySCENIC to find the top regulators (regulon) of cluster genes. As a result, it was confirmed that SOX4/SMARCA4, STAT1/2, and THRB/RAX were top regulator genes (master regulators) in the neural cell cluster (Neu1), the astrocyte cluster (Ast1), and the tanycyte-like cell cluster (Tan) (Figure 17).

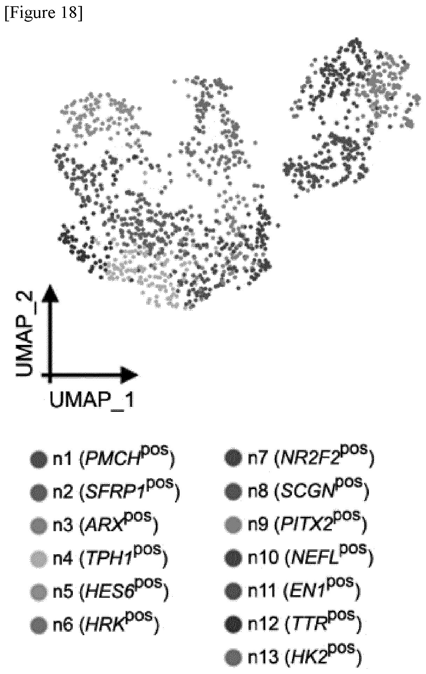

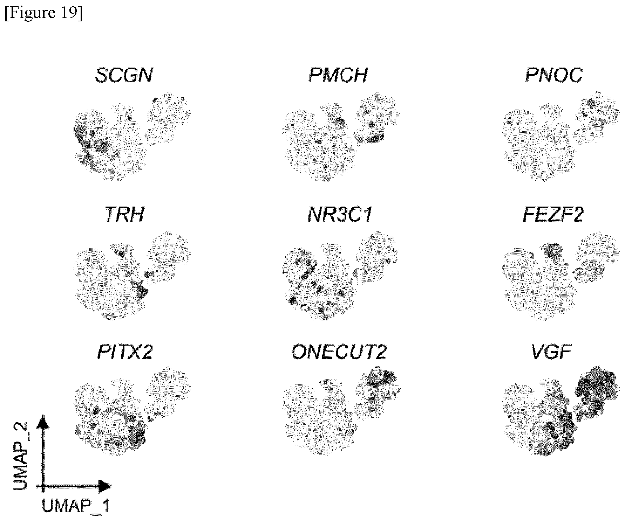

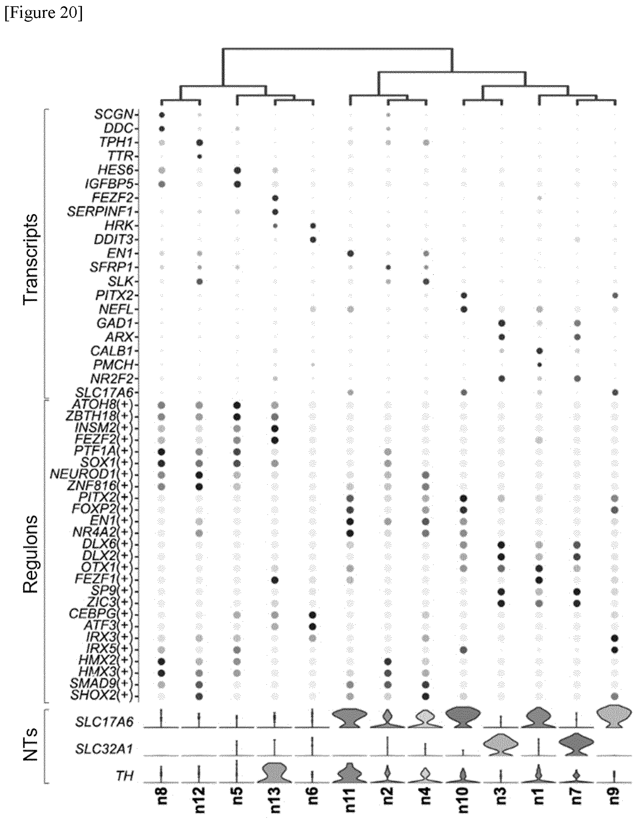

In order to further subdivide the above neural cell cluster (Neu1 and Neu2), additional clustering was performed. First, the above neural cell cluster (Neu1 and Neu2) was re-clustered to distinguish each cell cluster based on the most expressed gene in the respective cell cluster (Figure 18). As a result, a total of 13 cell clusters were clustered, and it was confirmed that most of the genes expressed in each cell cluster were related to hypothalamus development. For example, PITX2 is a gene specifically expressed during hypothalamus development and is an essential gene for hypothalamus development (PMID: 18206388). SCGN neural cells are hypothalamus specific neural cells that serve as gatekeepers for CRH-mediated stress responses (Figure 19). In addition, transcripts, transcription regulators (regulon), and neurotransmitters (NTs) expressed in each neural cell cluster were identified (Figure 20).

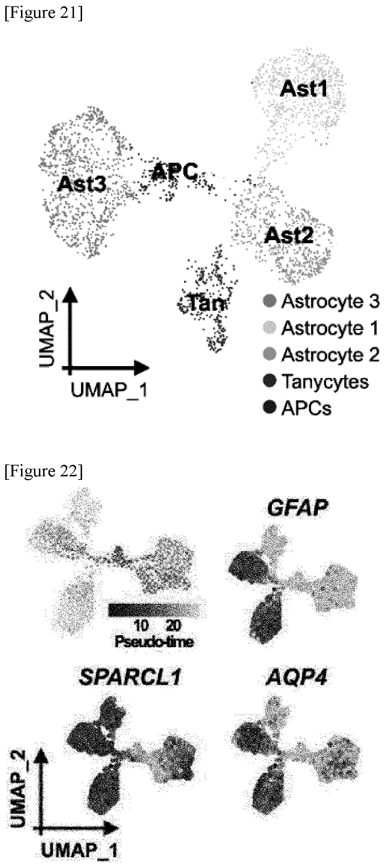

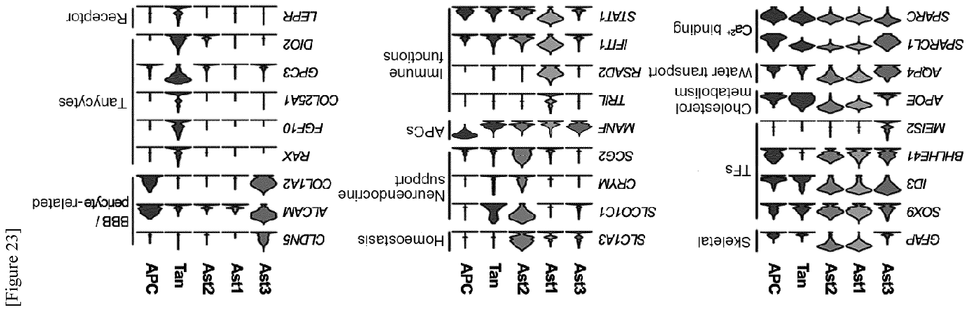

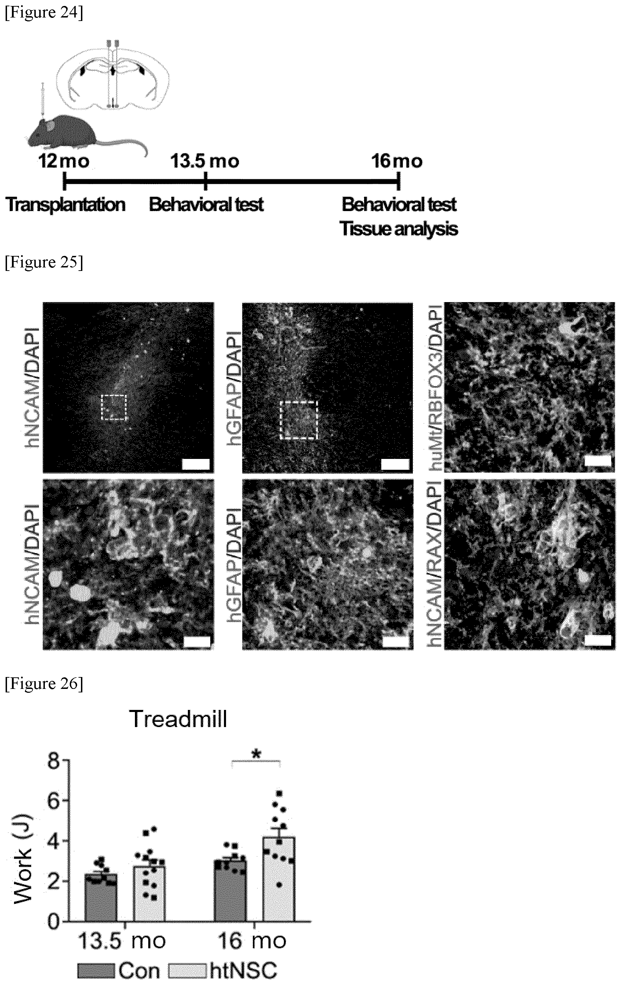

The astrocyte progenitor cell cluster (APC) was further clustered into three astrocyte clusters (Figures 21 to 23). As shown in Figure 23, it was confirmed that the Ast2 cluster was a cluster of astrocytes involved in homeostasis in which SLC1A3 (GLAST) was expressed, and it could mainly function to supply nutrients to neural cells. In addition, the Ast2 cluster was found to be related to neuroendocrine function based on the expression of genes such as SLCO1C1 (thyroid hormone transporter), CRYM (crystallin mu, a thyroid hormone binding protein), and SCG2 (secretogranin II, a neuroendocrine secretory protein). In the Ast1 cluster, it was confirmed that genes related to immune function such as RSAD2 and TRIL and genes induced by interferon (ISG5, IFIT, and STAT1) were specifically expressed. The Ast3 cluster differentiated from astrocyte progenitor cells (APC) expressed astrocyte-related transcription factors such as SOX9 and ID3, but not GFAP. In the Ast3 group, it was confirmed that Meis2, which is associated with the quiescence or activation of NCS in the subependymal zone, was expressed (PMID: 31304985). In addition, the Ast3 cluster expressed genes required for tight junction formation, such as CLDN5, ALCAM, and COL1A2 (Figure 24). In a recently reported study, it was confirmed that COL1A2 was expressed by a subset of astrocytes in the SVZ (PMID: 34413515, PMID: 30625322). Therefore, based on the above results, we hypothesized that the Ast3 cluster would be "type B" astrocytes that interact with brain parenchymal cells and/or pericytes. In addition, since it was recently reported that qNSCs exist in the subventricular layer of the third ventricle of the hypothalamus, it was assumed that the Ast3 cluster was qNSC.

Until now, the developmental origin of tanycytes has not been confirmed. However, similar to the recent report that tanycytes originate from astrocyte populations (Romanov et al., 2020), it was confirmed in the present invention that the tanycyte-like cell (Tan) cluster originated from the Ast2 cluster based on the single cell RNA sequencing analysis results (Figures 14 and 23).

In the case of the tanycyte (Tan) cluster, the expression of tanycyte specific marker genes such as RAX, FGF10, COL25A1, CRYM, and SCN7A was confirmed. In addition, since the expression of LEPR (leptin receptor) was confirmed, it could be expected that tanycytes could be involved in metabolic homeostasis through leptin signaling.

Through the above single cell RNA sequencing analysis, it was confirmed that the neural cell differentiation method of organoid-derived human htNSCs was similar to the major hypothalamus cell population *in vivo.* In addition, it was confirmed that tanycytes and hypothalamic qNSCs could be produced through the differentiation method of the present invention.

**Experimental Result 3. Confirmation of the effect of ameliorating aging in mice transplanted with organoid-derived human htNSCs**

A previous study reported that when mouse-derived htNSCs were transplanted into the aging mice, the rate of physiological changes related to aging was reduced (Zhang et al., 2017). Therefore, in order to confirm the anti-aging effect of hypothalamus-like organoid-derived human htNSCs produced from the hESCs of the present invention, the htNSCs were transplanted into 12-month-old aging mice (Figure 24).

The human htNSCs were transplanted twice into the mediobasal hypothalamus (MBH) region and followed up for 1 to 4 months. At this time, the mice injected with vehicle were used as a control group. As a result, cells expressing the human specific marker hNCAM (hNCAM+) were observed in the hypothalamus of mice 1 month after transplantation. The expression of RAX, an htNSC specific marker protein, was observed in some of the hNCAM+ cells, and the expression of RBFOX3 or human GFAP (hGFAP) protein was also observed (Figure 25).

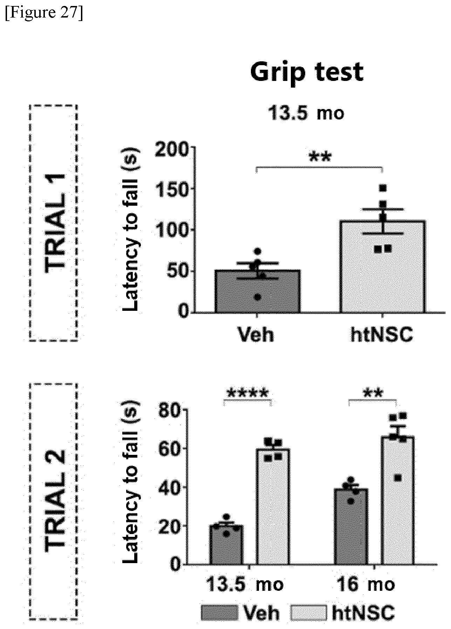

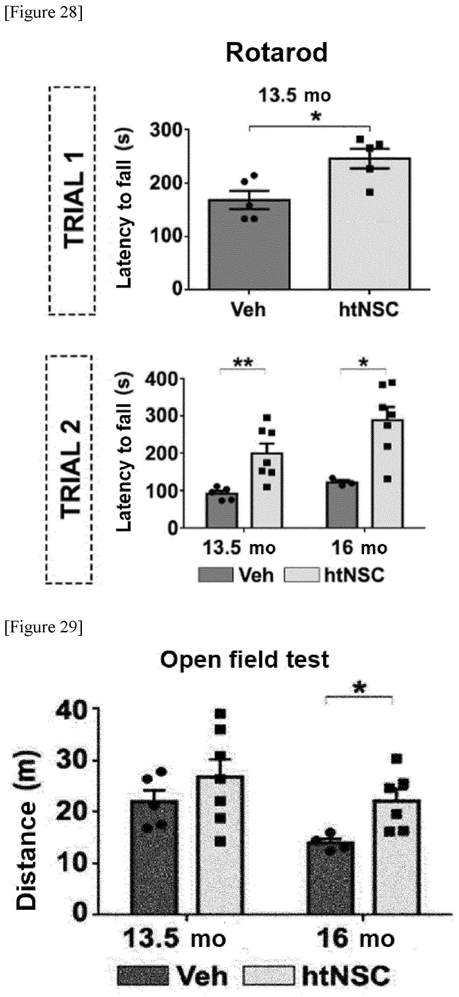

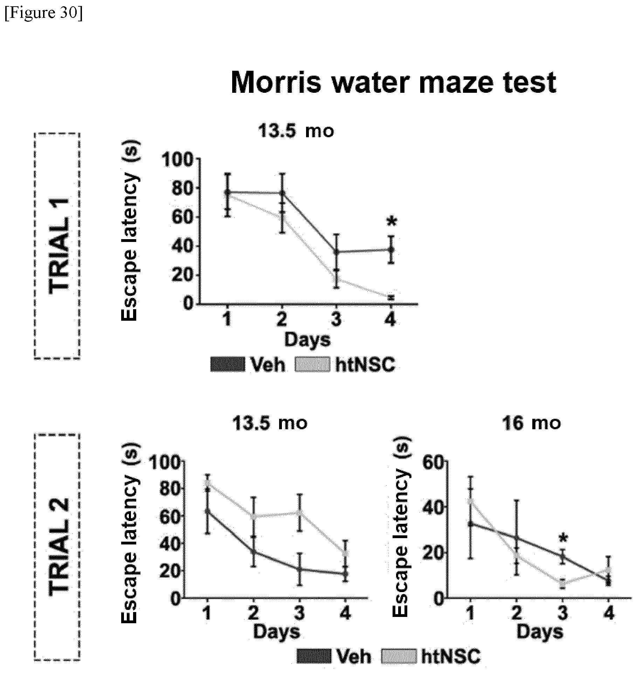

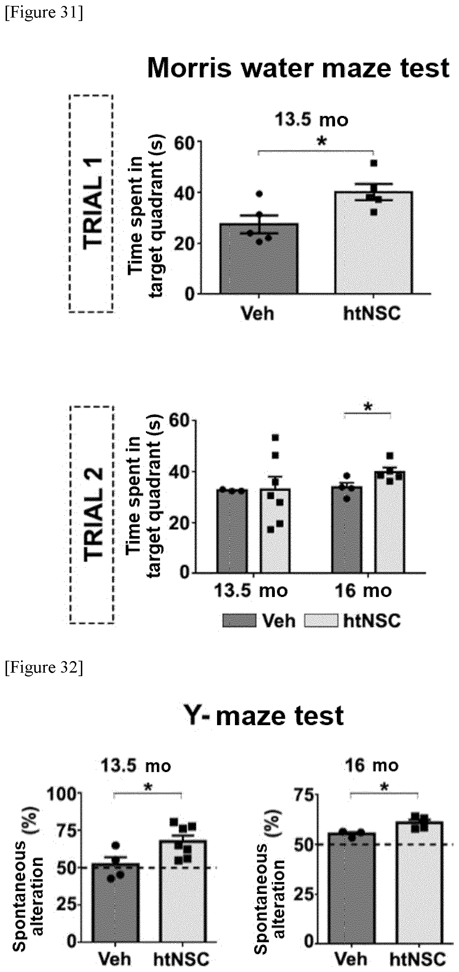

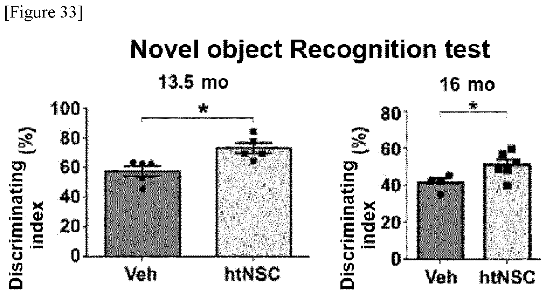

According to the results of the mouse behavior experiment, the endurance (treadmill performance) (Figure 26) of the human htNSC transplanted group was improved compared to the control group 4 months after transplantation. In addition, the muscle endurance (grip) (Figure 27), motor coordination (rotoarod) (Figure 28), and activity (locomotor activity, open field test) (Figure 29) of the human htNSC transplanted group were improved compared to the control group 1.5 months and 4 months after transplantation. In addition, the long-term and spatial memory and cognitive ability (Morris water maze test, Figures 30 and 31) (Y maze test, Figure 32) (novel object recognition test, Figure 33) of the human htNSC transplanted group were also improved compared to the control group.

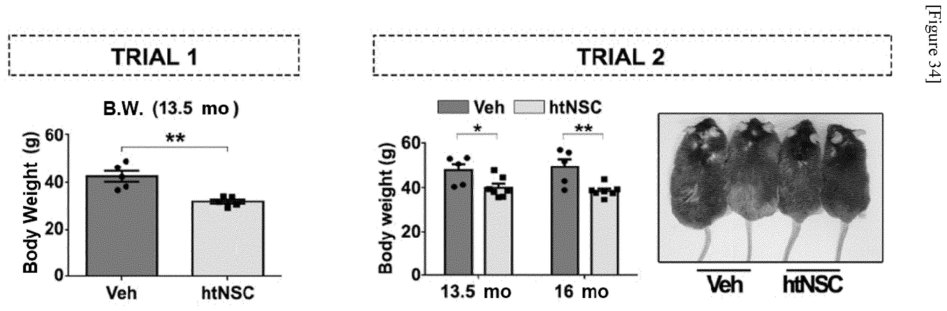

The hypothalamus regulates systemic metabolism by regulating neurotransmission through endocrine substances such as orexin and TRH as well as the sympathetic autonomic nervous system. It has been reported that this regulatory function of the hypothalamus is impaired as aging progresses (Waterson et al., 2015, Saper et al., 2014, Gonzalez-Garcia et al., 2020). The human htNSC transplanted group of the present invention suppressed systemic body metabolism and tissue aging due to aging compared to the control group. As shown in Figure 34, when the body weight of the mice was observed 1.5 months (13.5 months old) and 4 months (16 months old) after transplanting human htNSCs, a significant weight loss was observed in the human htNSC transplanted group compared to the body weight of the control group (Figure 34).

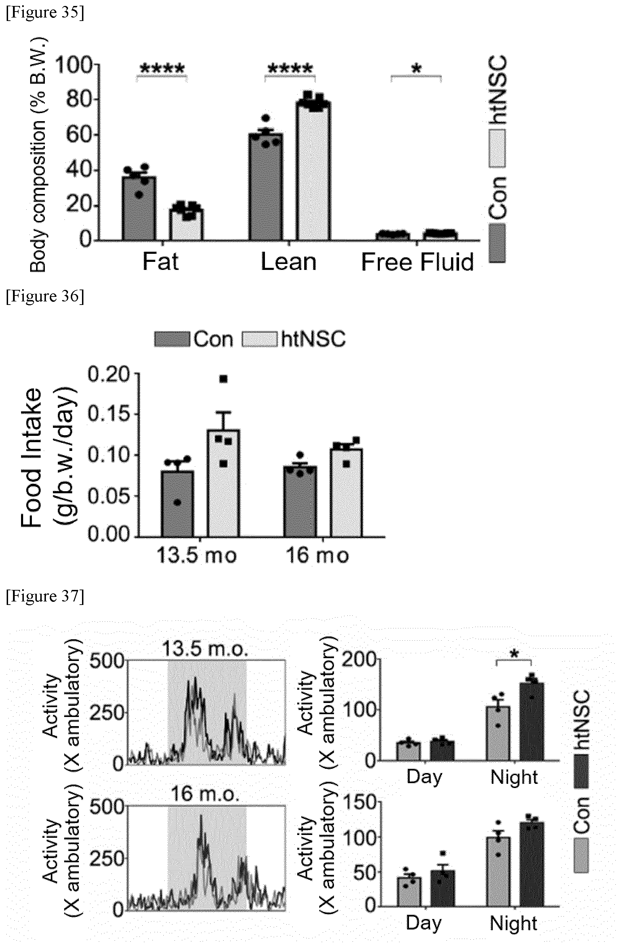

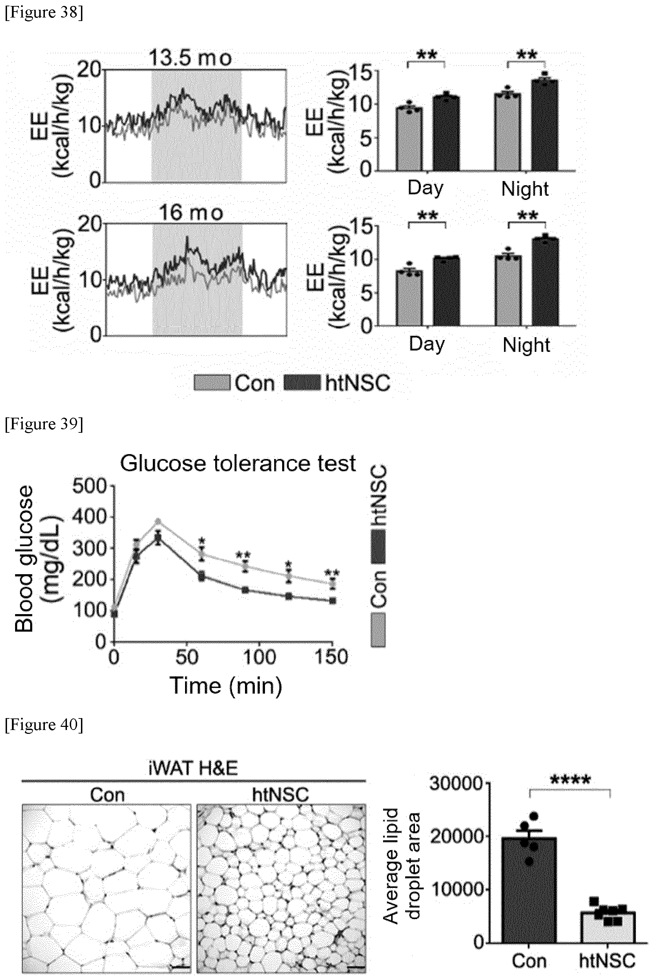

In addition, fat% was reduced compared to the control group, while lean% was significantly increased (Figure 35). In contrast, there was no significant difference in food intake between the control group and the human htNSC transplanted group. As shown in Figure 36, the results of the metabolic cage experiment showed that the locomotor activity (during nighttime 1.5 months (13.5 months old) after transplantation) of the human htNSC transplanted group was significantly increased compared to the control group (Figure 37). In addition, after 1.5 months (13.5 months old) or 4 months (16 months old) after transplantation, the energy expenditure (EE) was significantly increased in the human htNSC transplanted group compared to the control group both during nighttime and during daytime (Figure 38). In addition, 4 months after transplantation of human htNSCs, the glucose tolerance of the human htNSC transplanted group was significantly improved compared to the control group (Figure 39).

It is known that as aging progresses, not only body fat increases but also the amount and activity of brown adipose tissue (BAT) decrease. In order to confirm the effect of htNSCs of the present invention on the above-mentioned aging phenomenon, 4 months (16 months old) after transplantation of human htNSCs, inguinal white adipose tissue (iWAT), epididymal white adipose tissue (eWAT), and interscapular brown adipose tissue (iBAT) were stained with H&E and each tissue was observed.

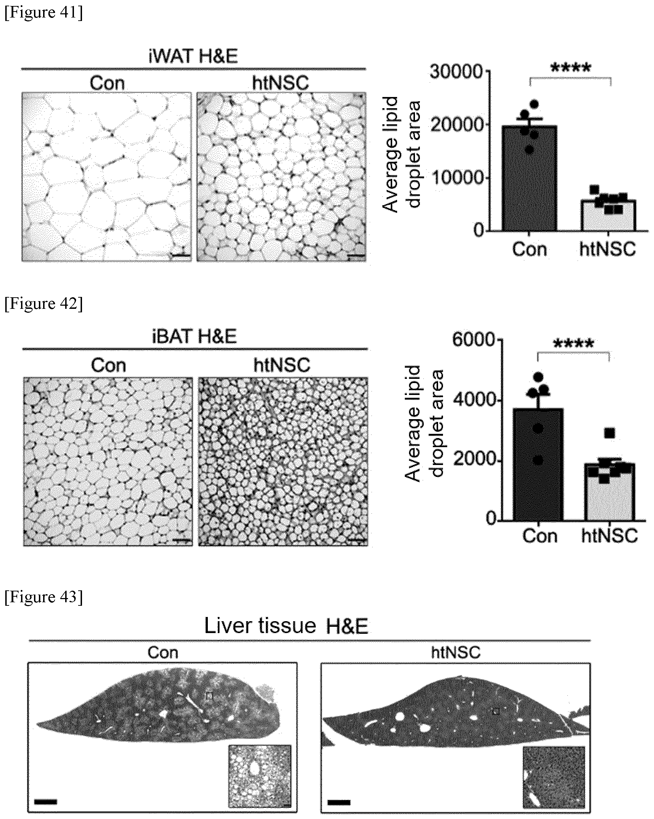

As a result, the size of adipocytes in the white adipose tissue and brown adipose tissue of the human htNSC transplanted group was significantly reduced compared to the control group (Figures 40 to 42). In addition, while lipid accumulation is increased with aging in the liver tissue, and lipid accumulation in the liver tissue of the htNSC transplanted group was reduced compared to the control group (Figure 43).

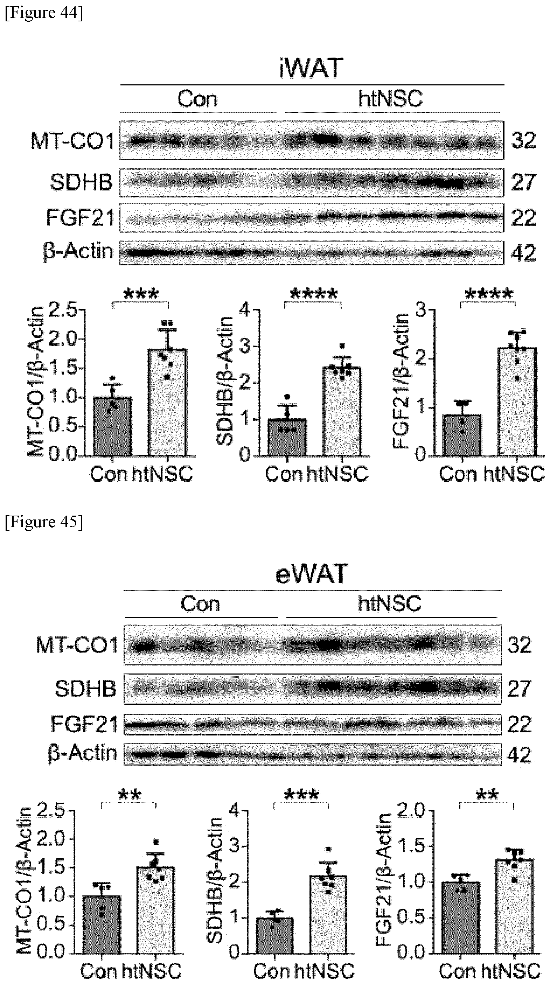

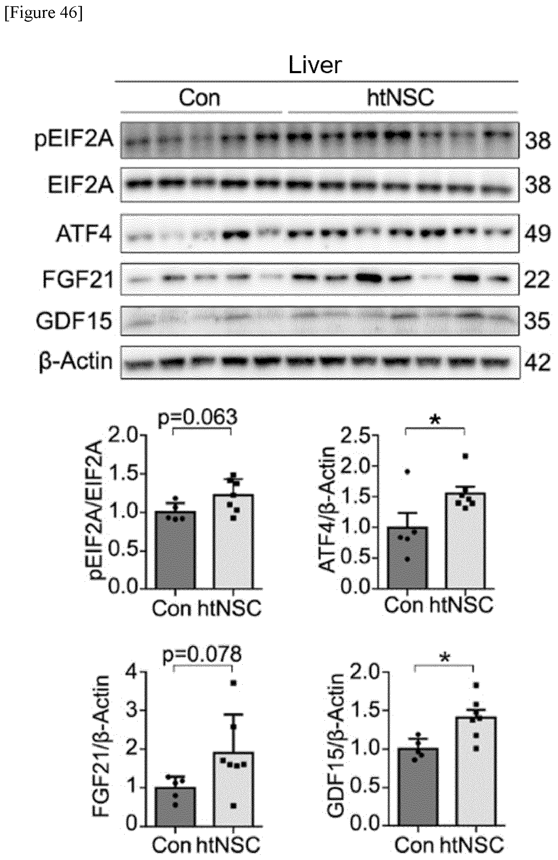

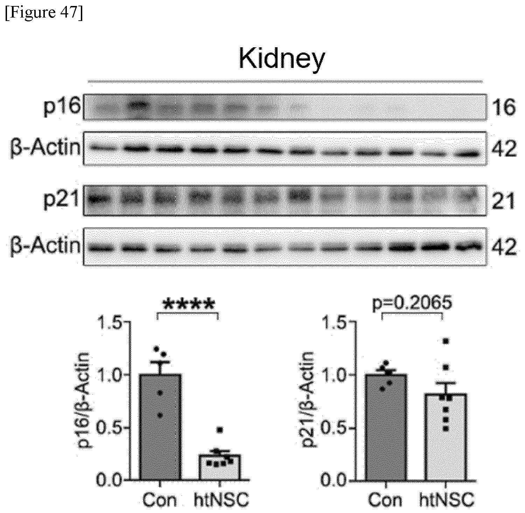

In white adipose tissue (iWAT and eWAT), the expression of MTCO1 and SDHB, which are mitochondrial homeostasis genes related to browning, and the expression of FGF21, a major regulator of brown adipocyte differentiation and energy metabolism regulation, were significantly increased (Figures 44 and 45). In addition, the expression of FGF21 and GDF15, and the phosphorylation of the transcription factor eIF2A induced by stress and the expression of ATF4 were increased in the liver tissue of the transplanted group (Figure 46). In addition, the expression of p16 and p21 proteins related to cell aging was reduced in the kidneys of the htNSC transplanted group compared to the control group (Figure 47).

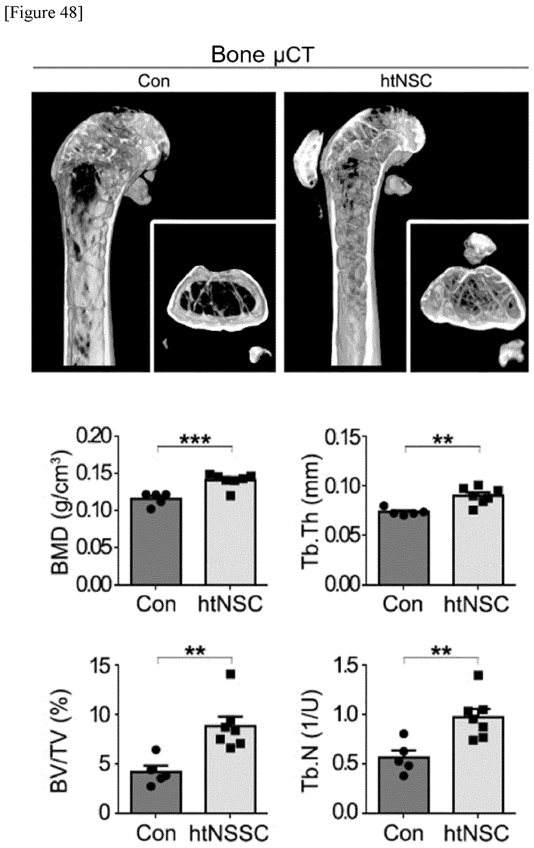

The hypothalamus is known to regulate not only systemic metabolism but also bone metabolism (remodeling and homeostasis) (Sharan et al., 2014). In the present invention, examination of the femur by X-ray at 4 months (16 months old) after transplantation of htNSCs into 12-month-old mice, revealed that while osteoporosis was observed in the control group, the bone mineral density (BMD), bone volume (BV)/tissue volume (TV) ratio, trabecular thickness (Tb.Th), and trabecular number (Tb.N) indices were significantly increased in the htNSC transplanted group compared to the control group (Figure 48).

**Experimental Result 4. Confirmation of anti-aging effect through intraventricular transplantation of htNSC-derived exosomes**

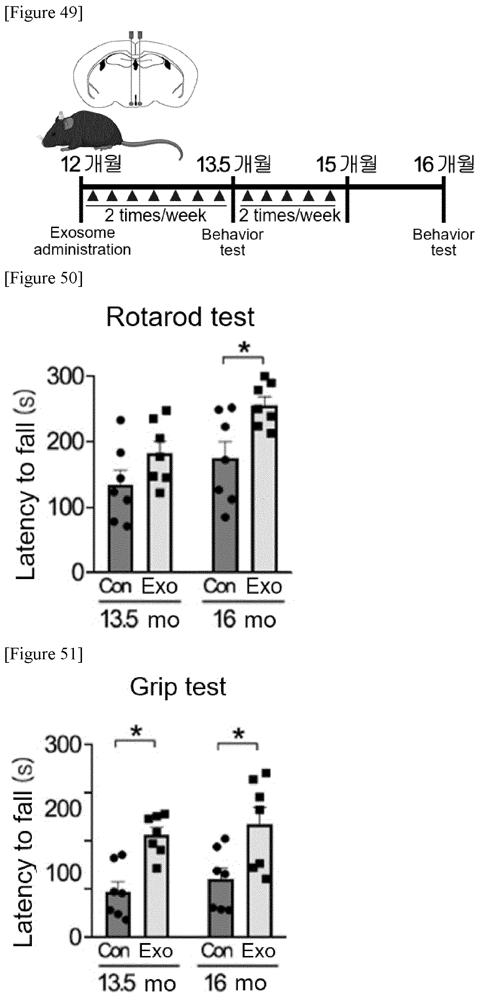

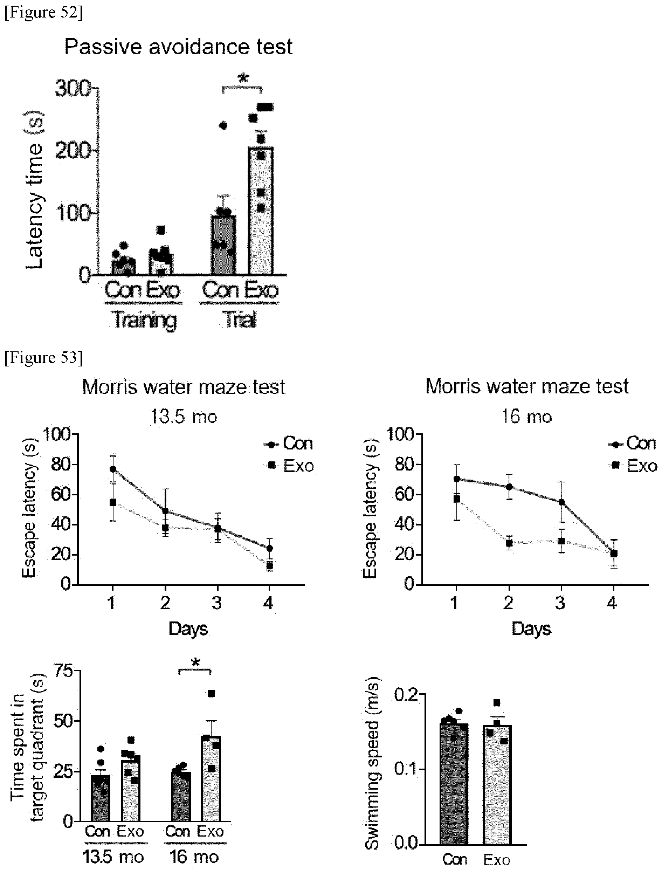

Recent studies have reported that exosomes secreted from transplanted NSCs exhibit therapeutic effects (Zhang et al., 2017). Therefore, in order to confirm the anti-aging effect of human htNSC-derived exosomes, exosomes were isolated from the culture fluid of human htNSCs derived from human hypothalamus organoids and injected into the third ventricle of 12-month-old mice using an osmotic pump (Figure 49). As a result, similar to the results of the human htNSC transplantation experiment above, injection of the htNSC-derived exosomes showed increase in motor coordination (rotarod) and muscle strength (grip) 1.5 months and 4 months after injection, respectively, compared to the control group (Figures 50 and 51). In addition, it was confirmed that the memory of mice injected with htNSC-derived exosomes [(passive avoidance test, Figure 52) and (Morris water maze test, Figure 53)] was improved compared to the control group.

Based on the above results, it was confirmed that human htNSC-derived exosomes can be potentially utilized as a method for preventing and treating aging.

**Claims**

1. A pharmaceutical composition for preventing and treating geriatric diseases, comprising hypothalamus-like organoid-derived neural stem cells as an active ingredient.

2. The pharmaceutical composition for preventing and treating geriatric diseases according to claim 1, wherein the hypothalamus-like organoid-derived neural stem cell expresses any one neural stem cell marker selected from the group consisting of SOX2, NESTIN, and KI67, and expresses any one hypothalamus marker selected from the group consisting of RAX, NKX2-1, ISL1, OTP, and SF-1.

3. The pharmaceutical composition for preventing and treating geriatric diseases according to claim 2, wherein the hypothalamus-like organoid-derived neural stem cell expresses the neural stem cell marker and the hypothalamus marker at 84% or more in the entire DAPI-positive cell population.

4. The pharmaceutical composition for preventing and treating geriatric diseases according to claim 1, wherein the hypothalamus-like organoid-derived neural stem cell is capable of proliferating for 1 to 10 passages.

5. The pharmaceutical composition for preventing and treating geriatric diseases according to claim 1, wherein the hypothalamus-like organoid-derived neural stem cell is differentiated into a neural cell that expresses at least one neural cell marker selected from the group consisting of synapsin1 (SYN1), TuJ1 (TUBB), MAP2, and RBFOX3 (NeuN), and expresses at least one hypothalamus neuropeptide selected from the group consisting of $\alpha$-MSH, neuropeptide Y (NPY), secretagogin (SCGN), and PMCH.

6. The pharmaceutical composition for preventing and treating geriatric diseases according to claim 5, wherein the neural cell is responsive to leptin and ghrelin.

7. The pharmaceutical composition for preventing and treating geriatric diseases according to claim 1, wherein the hypothalamus-like organoid-derived neural stem cell is differentiated and clustered into a neural stem/progenitor cell cluster (NSC), an intermediate progenitor cell cluster (IPC), a neural cell cluster (Neu1 and Neu2), an astrocyte progenitor cell cluster (APC), an astrocyte cluster (Ast1, Ast2 and Ast3), a tanycyte cluster (Tan), an interneuron progenitor cell cluster (Int1 and Int2), a radial glia cluster (RG), and a proteoglycan-secreting glia cluster.

8. The pharmaceutical composition for preventing and treating geriatric diseases according to claim 7, wherein the neural cell cluster (Neu1 and Neu2) is **characterized in that** the expression of genes related to hypothalamus development is increased.

9. The pharmaceutical composition for preventing and treating geriatric diseases according to claim 7, wherein the astrocyte progenitor cell cluster is **characterized in that** it is differentiated into

   a cell cluster (Ast2) expressing at least one gene selected from the group consisting of SLC1A3, SLCO1C1, CRYM, and SCG2;
   a cell cluster (Ast1) expressing at least one gene selected from the group consisting of RSAD2, TRAIL, ISG5, IFITs, and STAT1; and
   a cell cluster (Ast3) expressing at least one gene selected from the group consisting of SOX9, ID3, Meis2, CLDN5, ALCAM, and COL1A2.

10. The pharmaceutical composition for preventing and treating geriatric diseases according to claim 7, wherein the tanycyte cluster (Tan) expresses at least one gene selected from the group consisting of RAX, FGF10, COL25A1, CRYM, SCN7A, and LEPR.

11. The pharmaceutical composition for preventing and treating geriatric diseases according to claim 1, wherein the geriatric disease is any one selected from the group consisting of a neurodegenerative disease, osteoporosis, and a muscle aging-related disease.

12. The pharmaceutical composition for preventing and treating geriatric diseases according to claim 11, wherein the neurodegenerative disease is any one selected from the group consisting of Parkinson's disease, dementia, Alzheimer's disease, Huntington's disease, amyotrophic lateral sclerosis, memory loss, myasthenia gravis, progressive supranuclear palsy, multiple system atrophy, essential tremor, corticobasal degeneration, diffuse Lewy body disease, and Pick disease.

13. The pharmaceutical composition for preventing and treating geriatric diseases according to claim 11, wherein the muscle aging-related disease is selected from the group consisting of muscular atrophy, muscle disuse atrophy, and senile sarcopenia.

14. An exosome secreted from a hypothalamus-like organoid-derived neural stem cell.

15. The exosome according to claim 14, wherein the hypothalamus-like organoid-derived neural stem cell expresses any one neural stem cell marker selected from the group consisting of SOX2, NESTIN, and KI67, and expresses any one hypothalamus marker selected from the group consisting of RAX, NKX2-1, ISL1, OTP, and SF-1.

16. The exosome according to claim 14, wherein the hypothalamus-like organoid-derived neural stem cell expresses the neural stem cell marker and the hypothalamus marker at 84% or more in the entire DAPI-positive cell population.

17. A pharmaceutical composition for preventing and treating geriatric diseases, comprising the exosomes according to claim 14 as an active ingredient.

18. The pharmaceutical composition according to claim 17, wherein the geriatric disease is a neurodegenerative disease and a muscle aging-related disease.

19. The pharmaceutical composition according to claim 18, wherein the neurodegenerative disease is selected from the group consisting of Parkinson's disease, dementia, Alzheimer's disease, Huntington's disease, amyotrophic lateral sclerosis, memory loss, myasthenia gravis, progressive supranuclear palsy, multiple system atrophy, essential tremor, corticobasal degeneration, diffuse Lewy body disease, and Pick disease.

20. The pharmaceutical composition according to claim 18, wherein the muscle aging-related disease is selected from the group consisting of muscular atrophy, muscle disuse atrophy, and senile sarcopenia.

21. A method for producing an exosome, comprising the steps of:

    i) culturing hypothalamus-like organoid-derived neural stem cells; and
    ii) collecting exosomes from the culture fluid.

22. A kit for preventing and treating geriatric diseases, comprising the hypothalamus organoid-derived neural stem cells according to claim 1; and/or the exosomes according to claim 14.

23. The kit according to claim 22, wherein the hypothalamus-like organoid-derived neural stem cell expresses any one neural stem cell marker selected from the group consisting of SOX2, NESTIN, and KI67, and expresses any one hypothalamus marker selected from the group consisting of RAX, NKX2-1, ISL1, OTP, and SF-1.

24. The kit according to claim 22, wherein the hypothalamus-like organoid-derived neural stem cell expresses the neural stem cell marker and the hypothalamus marker at 84% or more in the entire DAPI-positive cell population.

25. A use of the hypothalamus organoid-derived neural stem cell according to claim 1; or the exosome according to claim 14 for the prevention or treatment of geriatric diseases.

26. A method for preventing or treating geriatric diseases, comprising administering to a subject the hypothalamus organoid-derived neural stem cell according to claim 1; or the exosome according to claim 14.

[Figure 1]

[Figure 2]

[Figure 3]

[Figure 4]

[Figure 5]

[Figure 6]

[Figure 7]

[Figure 8]

[Figure 9]

[Figure 10]

[Figure 11]

[Figure 12]

[Figure 13]

[Figure 14]

[Figure 15]

[Figure 16]

[Figure 17]

[Figure 18]

n1 (*PMCH*^pos)  n7 (*NR2F2*^pos)

n2 (*SFRP1*^pos)  n8 (*SCGN*^pos)

n3 (*ARX*^pos)  n9 (*PITX2*^pos)

n4 (*TPH1*^pos)  n10 (*NEFL*^pos)

n5 (*HES6*^pos)  n11 (*EN1*^pos)

n6 (*HRK*^pos)  n12 (*TTR*^pos)

n13 (*HK2*^pos)

[Figure 19]

[Figure 20]

[Figure 21]

[Figure 22]

[Figure 23]

[Figure 24]

12 mo      13.5 mo      16 mo

Transplantation    Behavioral test      Behavioral test
Tissue analysis

[Figure 25]

[Figure 26]

Treadmill

[Figure 27]

**Grip test**

[Figure 28]

Rotarod

[Figure 29]

Open field test

[Figure 30]

# Morris water maze test

[Figure 31]

## Morris water maze test

[Figure 32]

## Y- maze test

[Figure 33]

Novel object Recognition test

[Figure 34]

[Figure 35]

[Figure 36]

[Figure 37]

[Figure 38]

[Figure 39]

[Figure 40]

[Figure 41]

iWAT H&E

[Figure 42]

iBAT H&E

[Figure 43]

Liver tissue H&E

[Figure 44]

[Figure 45]

[Figure 46]

[Figure 47]

Kidney

[Figure 48]

Bone μCT

[Figure 49]

12 개월     13.5 개월     15 개월     16 개월

2 times/week    2 times/week

Exosome administration

Behavior test

Behavior test

[Figure 50]

## Rotarod test

[Figure 51]

## Grip test

[Figure 52]

## Passive avoidance test

[Figure 53]

## Morris water maze test
### 13.5 mo

## Morris water maze test
### 16 mo

| | INTERNATIONAL SEARCH REPORT | International application No. |
|---|---|---|
| | | **PCT/KR2023/006861** |

**A. CLASSIFICATION OF SUBJECT MATTER**

**A61K 35/30**(2006.01)i; **A61P 25/28**(2006.01)i; **A61P 19/10**(2006.01)i; **A61P 21/00**(2006.01)i; **A61P 43/00**(2006.01)i; **C12N 5/0797**(2010.01)i

According to International Patent Classification (IPC) or to both national classification and IPC

**B. FIELDS SEARCHED**

Minimum documentation searched (classification system followed by classification symbols)

A61K 35/30(2006.01); C12N 5/02(2006.01); C12N 5/0797(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 시상하부(hypothalamus), 오가노이드(organoid), 신경줄기세포(neural stem cells), 엑소좀(exosome), 노화(aging), 신경 퇴행성 질환(Neurodegenerative disease), 근육(muscle)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2021-0106966 A (CORESTEM CO., LTD.) 31 August 2021 (2021-08-31)<br>See claims 10 and 15, paragraphs [0009] and [0251]-[0256], and figures 37-40. | 1-6,11-25 |
| A | | 7-10 |
| Y | ZHANG, Y. et al. Hypothalamic stem cells control ageing speed partly through exosomal miRNAs. Nature. 2017, vol. 548, no. 7665, pp. 52-57 (published online: 26 July 2017).<br>See page 56, right column, METHODS. | 1-6,11-25 |
| A | QIAN, X. et al. Brain-Region-Specific Organoids Using Mini-bioreactors for Modeling ZIKV Exposure. Cell. 2016, vol. 165, no. 5, pp. 1238-1254 (published online: 22 April 2016).<br>See page 1246. | 1-25 |
| A | CN 112011512 A (ZHANG, Wenhua) 01 December 2020 (2020-12-01)<br>See claims 1-7. | 1-25 |

☑ Further documents are listed in the continuation of Box C.     ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **01 September 2023** | **01 September 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

| INTERNATIONAL SEARCH REPORT | International application No. |
|---|---|
| | **PCT/KR2023/006861** |

**C.** **DOCUMENTS CONSIDERED TO BE RELEVANT**

| Category* | Citation of document, with indication, where appropriate, of the relevant passages | Relevant to claim No. |
|---|---|---|
| A | KR 10-2007-0032311 A (THE UNIVERSITY COURT, THE UNIVERSITY OF EDINBURGH) 21 March 2007 (2007-03-21)<br>See claims 55 and 56, and paragraph [0134]. | 1-25 |

Form PCT/ISA/210 (second sheet) (July 2022)

<table>
<tr><td>INTERNATIONAL SEARCH REPORT</td><td>International application No.<br>**PCT/KR2023/006861**</td></tr>
</table>

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

   a.  ☑ forming part of the international application as filed.

   b.  ☐ furnished subsequent to the international filing date for the purposes of international search (Rule 13*ter*.1(a)),

   ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

| INTERNATIONAL SEARCH REPORT | International application No. |
|---|---|
| | **PCT/KR2023/006861** |

**Box No. II        Observations where certain claims were found unsearchable (Continuation of item 2 of first sheet)**

This international search report has not been established in respect of certain claims under Article 17(2)(a) for the following reasons:

1. ☑  Claims Nos.: **26**
   because they relate to subject matter not required to be searched by this Authority, namely:

   Claim 26 pertains to a method for treatment of the human body by means of a composition (PCT Article 17(2)(a)(i) and PCT Rule 39.1(iv)).

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/KR2023/006861**

| Patent document cited in search report | | | Publication date (day/month/year) | Patent family member(s) | | | Publication date (day/month/year) |
|---|---|---|---|---|---|---|---|
| KR | 10-2021-0106966 | A | 31 August 2021 | KR | 10-2020-0043297 | A | 27 April 2020 |
| | | | | KR | 10-2296446 | B1 | 02 September 2021 |
| | | | | KR | 10-2468360 | B1 | 17 November 2022 |
| CN | 112011512 | A | 01 December 2020 | None | | | |
| KR | 10-2007-0032311 | A | 21 March 2007 | AU | 2005-252434 | A1 | 22 December 2005 |
| | | | | AU | 2005-252434 | B2 | 24 November 2011 |
| | | | | CA | 2569978 | A1 | 22 December 2005 |
| | | | | CA | 2569978 | C | 06 June 2017 |
| | | | | CN | 101124319 | A | 13 February 2008 |
| | | | | CN | 101124319 | B | 13 March 2013 |
| | | | | CN | 103146649 | A | 12 June 2013 |
| | | | | CN | 103146649 | B | 22 May 2018 |
| | | | | EP | 1758986 | A2 | 07 March 2007 |
| | | | | EP | 1758986 | B1 | 30 September 2015 |
| | | | | EP | 3000877 | A1 | 30 March 2016 |
| | | | | EP | 3000877 | B1 | 14 August 2019 |
| | | | | GB | 2414995 | A | 14 December 2005 |
| | | | | IL | 179919 | B | 27 September 2011 |
| | | | | JP | 2008-501356 | A | 24 January 2008 |
| | | | | JP | 2012-034706 | A | 23 February 2012 |
| | | | | US | 2006-0014281 | A1 | 19 January 2006 |
| | | | | US | 2014-0370596 | A1 | 18 December 2014 |
| | | | | US | 8785187 | B2 | 22 July 2014 |
| | | | | US | 9309495 | B2 | 12 April 2016 |
| | | | | WO | 2005-121318 | A2 | 22 December 2005 |
| | | | | WO | 2005-121318 | A3 | 23 March 2006 |
| | | | | WO | 2005-121318 | A8 | 28 August 2014 |

Form PCT/ISA/210 (patent family annex) (July 2022)

## EP 4 527 395 A1

### REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WATAYA T et al.** *Proc Natl Acad Sci USA*, 2008, vol. 105 (33), 11796-11801
- **MERKLE FT et al.** *Development*, 2015, vol. 142 (4), 633-643
- Remington's Pharmaceutical Sciences. 1995